(19) **Europäisches Patentamt / European Patent Office / Office européen des brevets**

(11) **EP 3 234 173 B1**

(12) **FASCICULE DE BREVET EUROPEEN**

(45) Date de publication et mention de la délivrance du brevet:
**18.08.2021 Bulletin 2021/33**

(21) Numéro de dépôt: **15808767.6**

(22) Date de dépôt: **30.11.2015**

(51) Int Cl.:
*C12Q 1/18* (2006.01)   *C12Q 1/04* (2006.01)
*G16B 99/00* (2019.01)   *C12Q 1/06* (2006.01)
*G01N 33/569* (2006.01)

(86) Numéro de dépôt international:
**PCT/FR2015/053258**

(87) Numéro de publication internationale:
**WO 2016/097518 (23.06.2016 Gazette 2016/25)**

(54) **PROCÉDÉ ET DISPOSITIF DE CARACTÉRISATION DU POUVOIR INHIBITEUR D'UNE MOLÉCULE SUR UN MICROORGANISME**

VERFAHREN UND VORRICHTUNG ZUR CHARAKTERISIERUNG DER INHIBITORISCHEN KAPAZITÄT EINES MOLEKÜLS AUF EINEN MIKROORGANISMUS

METHOD AND DEVICE FOR CHARACTERIZING THE INHIBITORY CAPACITY OF A MOLECULE ON A MICROORGANISM

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **15.12.2014 FR 1462408**

(43) Date de publication de la demande:
**25.10.2017 Bulletin 2017/43**

(73) Titulaire: **bioMérieux**
**69280 Marcy-l'Étoile (FR)**

(72) Inventeurs:
• **TOURNOUD, Maud**
  **38000 Grenoble (FR)**
• **MAHE, Pierre**
  **38250 Lans en Vercors (FR)**

(74) Mandataire: **Le Mauff, Frédéric Francis Joseph et al**
**bioMérieux**
**376, chemin de l'Orme**
**69280 Marcy-l'Étoile (FR)**

(56) Documents cités:
EP-A1- 0 010 846   WO-A1-2008/078911
WO-A1-2012/073202   WO-A1-2014/155020
WO-A2-2005/021559   WO-A2-2008/107881
FR-A1- 2 916 761   US-A1- 2012 077 206

• **None**

**Description**

**DOMAINE DE L'INVENTION**

L'invention a trait au domaine de l'analyse du pouvoir inhibiteur d'une molécule sur la croissance d'un microorganisme, et notamment le pouvoir inhibiteur d'un antibiotique sur la croissance d'une bactérie et le pouvoir inhibiteur d'un antifongique sur la croissance d'une levure ou d'une moisissure.

**ETAT DE LA TECHNIQUE**

L'activité d'un antibiotique sur une bactérie est caractérisée notamment par la « concentration minimale inhibitrice », ou CMI, qui se définit comme la concentration minimale de l'antibiotique à mélanger à une population de la bactérie pour inhiber en totalité sa multiplication.

Une première technique employée pour mesurer la CMI consiste, pour un technicien de laboratoire, à préparer plusieurs tubes à paroi transparente comprenant une concentration initiale de la bactérie, concentration qui est identique pour tous les tubes, un milieu nutritif pour la bactérie, et une concentration initiale d'antibiotique qui est croissante en fonction de l'ordre des tubes disposés dans un rack. Une fois les tubes préparés avec le gradient de concentration d'antibiotique, le technicien met en alors en incubation les tubes. Si la concentration d'un antibiotique dans un tube est trop faible pour inhiber la croissance des bactéries, un trouble apparait dans le tube, ce trouble étant de plus en plus présent à mesure que la population bactérienne croît. Après une durée d'incubation donnée, le technicien de laboratoire inspecte donc visuellement les tubes et identifie la CMI comme étant égale à la concentration d'antibiotique la plus faible parmi les tubes qui ne montrent aucun trouble. On conçoit aisément l'imprécision d'une telle méthode. Non seulement le nombre de tubes préparés est très réduits, usuellement à moins de 10, ce qui ne permet pas d'obtenir une précision élevée sur la CMI, mais en outre l'identification de celle-ci dépend du jugement d'un technicien concernant la présence ou non d'un trouble dans un tube.

Des dispositifs et procédés ont donc été mis au point pour augmenter à la fois la précision sur la concentration d'antibiotique dans un échantillon et la robustesse de la détection d'une croissance bactérienne dans un échantillon. Plus particulièrement, des dispositifs sont aujourd'hui capables de préparer une grande quantité d'échantillons bactériens, pouvant chacun comprendre leur propre concentration d'antibiotique, et capables de mesurer automatiquement et avec précision une grandeur qui dépend de la taille de la population bactérienne dans un échantillon, par exemple une fluorescence ou une densité optique. Notamment, le dispositif décrit dans l'article « Millifluidic droplet analyser for microbiology » de L. Baraban et al, Lab on Chip, 2011, 11, 4057, produit un train de gouttelettes de volume inférieur au micro litre et dont la composition en bactéries, milieu nutritif et antibiotique peut être ajustée avec précision. Un tel dispositif permet notamment de produire un train de plusieurs centaines à plusieurs milliers de gouttelettes de volume constant et comprenant chacune un nombre initial fixe de bactéries, et une concentration initiale d'antibiotique qui est décroissante en fonction de la position des gouttelettes dans le train.

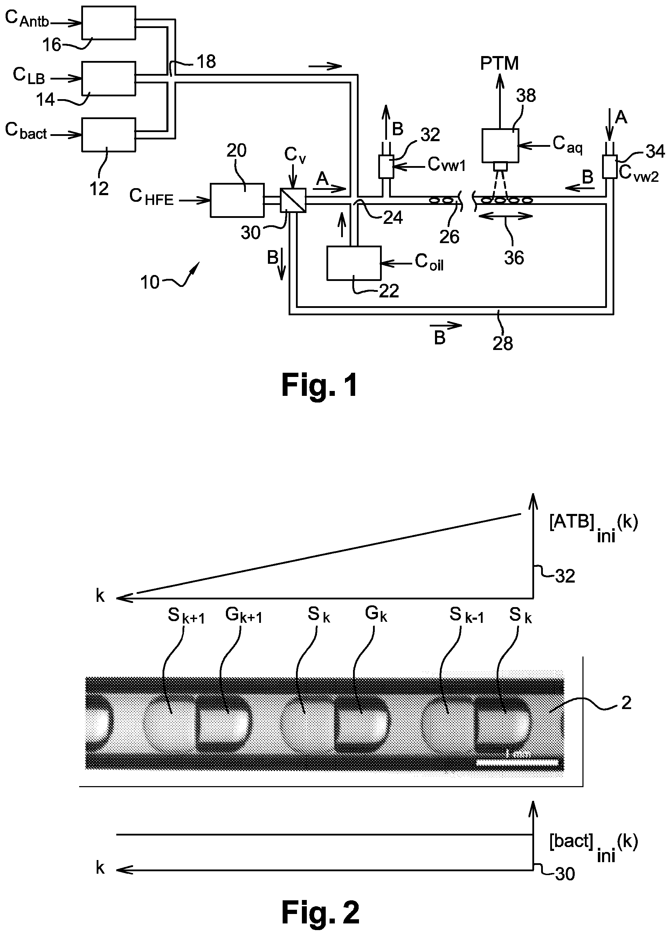

En se référant à la vue schématique de la figure 1, ce dispositif **10** comporte pour la production du train de gouttelettes :

- trois seringues **12, 14, 16,** dont le débit est pilotable et contenant respectivement une solution aqueuse de bactéries, une solution aqueuse de nutriments et une solution aqueuse d'antibiotique. Les seringues **12, 14, 16** injectent de manière contrôlée leurs contenus dans une jonction **18** où ils sont mélangés pour former une solution aqueuse ; et
- deux seringues **20, 22** dont le débit est pilotable et contenant respectivement une première et une seconde huiles, non miscibles avec la solution aqueuse, et non miscibles entre elles. Le contenu de ces seringues est injecté au niveau d'une jonction **24**. Cette jonction reçoit par ailleurs la solution aqueuse en provenance de la jonction **18** et débouche sur un tube principal transparent **26**. La première huile, par exemple du type hydrofluoroéther, notamment du HFE-7500®, sert de milieu continu dans lequel des gouttelettes de solution aqueuse en provenance de la jonction **18** sont déposées. La seconde huile, minérale, est utilisée pour former des éléments d'espacement des gouttelettes. Notamment, après chaque gouttelette de solution aqueuse formée dans la première huile, une gouttelette de la seconde huile est déposée dans la première huile.

En se référant à la figure 2, les seringues **12, 14, 16, 20, 22** sont donc pilotées par ordinateur afin d'obtenir un train uniforme, tant en volume qu'en espacement, de gouttelettes de solution bactérienne dans le tube **26**. Sur la figure 2 sont illustrées trois gouttelettes de solution bactérienne $G_{k-1}$, $G_k$ et $G_{k+1}$ formées successivement dans la première huile **28** et séparées les unes des autres par des gouttelettes d'espacement $S_{k-1}$, $S_k$, $S_{k+1}$ constituées de la seconde huile. On note notamment que les gouttelettes $G_{k-1}$, $G_k$ et $G_{k+1}$ sont naturellement référencées par leur position dans le train de gouttelettes, ou de manière équivalente par un numéro, ou index, entier *k*. L'entier « 1 » correspond ainsi à la gouttelette formée en premier dans le train objet de l'étude par l'analyseur. Le contenu des trois premières seringues **12, 14, 16** et leur commande par ordinateur permet d'obtenir des gouttelettes comprenant :

- une même concentration initiale de bactéries [*bact*]$_{ini}$(*k*) = *constante,* telle qu'illustré par la courbe **30,** ou un nombre initial $N_0$ constant de bactéries ;
- et une concentration initiale d'antibiotique [*ATB*]$_{ini}$(*k*) qui décroit en fonction de la position *k* de la gouttelette dans le train de gouttelette, comme cela est illustré par la courbe **32.**

Pour la partie détection de la population de bactérie dans les gouttelettes, le dispositif **10** comporte :

- un tube secondaire 28 en parallèle du tube principal **26,** une vanne pilotable **30** permettant d'injecter l'huile de la seringue **20** soit dans le tube **26** soit dans le tube secondaire **28,** et deux vannes **32, 34,** disposées respectivement en début et en fin du tube principal **26** et pilotables entre une position ouverte et une position fermée. Les vannes **30, 32, 34,** pilotées par ordinateur, permettent ainsi de définir deux écoulements « A » et « B » de sens opposés dans le tube principal **26,** et donc un mouvement de va-et-vient **36** des gouttelettes dans le tube transparent **26** ; et
- un système de détection **38,** disposé en face du tube **26,** pour mesurer la fluorescence des gouttelettes à une longueur d'onde particulière.

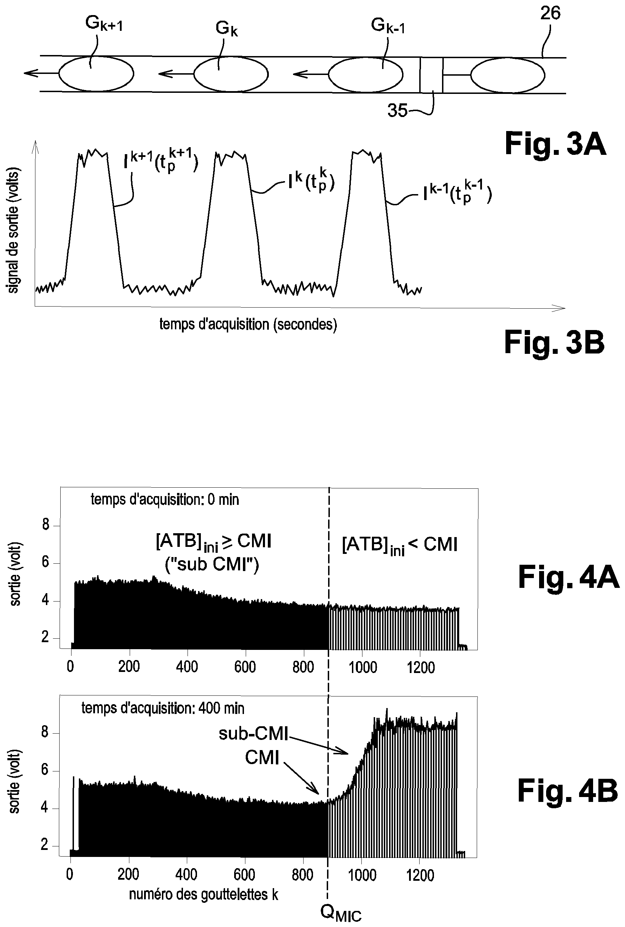

Notamment, les bactéries contenues dans les gouttelettes comprennent une molécule fluorescente, soit naturellement, soit artificiellement (par exemple par incorporation dans le génome de la bactérie d'un gène codant une protéine fluorescente). En variante, le milieu nutritif des gouttelettes comprend un élément métabolisable par les bactéries sous la forme d'une molécule fluorescente. La fluorescence d'une gouttelette dépend donc directement du nombre de bactéries contenues dans celle-ci. Le système de détection **38** comporte un ensemble d'éléments et circuits pour former un spot lumineux sur le tube principal **26** de manière à exciter la longueur d'onde de fluorescence des molécules fluorescentes, et mesurer la fluorescence induite par cette excitation. Ainsi, en se référant à la figure 3A, qui illustre un mouvement vers la gauche des gouttelettes G$_{k-1}$, G$_k$ et G$_{k+1}$, chacune d'entre elles passent dans le spot de détection **35** du système **38**. Le signal de mesure induit par le passage des gouttelettes G$_{k-1}$, G$_k$ et G$_{k+1}$ devant le système **28** produit un signal tel qu'illustré à la figure 3B, à savoir un train d'impulsions sensiblement sous la forme de créneaux $I^{k+1}\left(t_p^{k+1}\right), I^k\left(t_p^k\right), I^{k-1}\left(t_p^{k-1}\right).$

Le dispositif **10** permet un passage rapide de l'ensemble des gouttelettes dans le spot **35**. Le passage de toutes les gouttelettes dans le spot **35** peut prendre en effet moins d'une minute. La fréquence des passages devant le spot **35** et/ou la fréquence des mesures peut quant à elle être réglée indépendamment de la vitesse du mouvement de va-et-vient. Pour des bactéries par exemple, la fluorescence des gouttelettes est mesurée environs 8 fois par heure ou toutes les 7 à 8 minutes durant 2h à 16h environ.

En fonctionnement, le dispositif **10** produit donc un train de *N* gouttelettes dans le tube principal **26,** puis la seringue **20** et les vannes **30, 32,** sont pilotées afin de produire un mouvement de va-et-vient des gouttelettes dans le tube **26** afin que chacune des gouttelettes passe à intervalle régulier devant le système de détecteur **28** qui mesure sa fluorescence. Un cycle de mesure est ainsi constitué du passage de l'ensemble des gouttelettes dans le spot **35** pour leur mesure. Lors d'un cycle de mesure *« p »,* les gouttelettes sont mesurées à des instants différents, l'instant de mesure d'une gouttelette « k » étant égal à $t_p^k.$ L'instant $t_p$ est quant à lui l'instant de mesure de la dernière gouttelette lors du p$^{ième}$ cycle, c'est-à-dire l'instant de mesure $t_p^1$ ou $t_p^N$ en fonction du sens du va-et-vient lors du p$^{ième}$ cyle de mesure.

Le signal de mesure est ensuite traité par ordinateur pour réduire chaque impulsion mesurée $I^k\left(t_p^k\right)$ à une valeur $x^k\left(t_p^k\right),$ par exemple la moyenne du plateau de chaque impulsion, et les valeurs $x^k\left(t_p^k\right)$ ainsi produites sont mémorisées dans l'ordinateur avec leur instant d'acquisition $t_p^k.$ Les valeurs $x^k\left(t_p^k\right)$ sont donc représentatives de la quantité de bactéries contenues dans les gouttelettes. Pour chaque gouttelette *k*, il est donc produit un ensemble de mesures $\left\{x^k(t_1^k), x^k(t_2^k), \ldots, x^k(t_p^k), \ldots, x^k(t_P^k)\right\}$ correspondant à l'ensemble d'instants de mesures $\left\{t_1^k, t_2^k, \ldots, t_p^k, \ldots, t_P^k\right\}$ jusqu'à l'instant $t_P^k = t_p.$ Notamment, chaque instant d'acquisition $t_p^k$ correspond à une durée particulière d'incubation des gouttelettes.

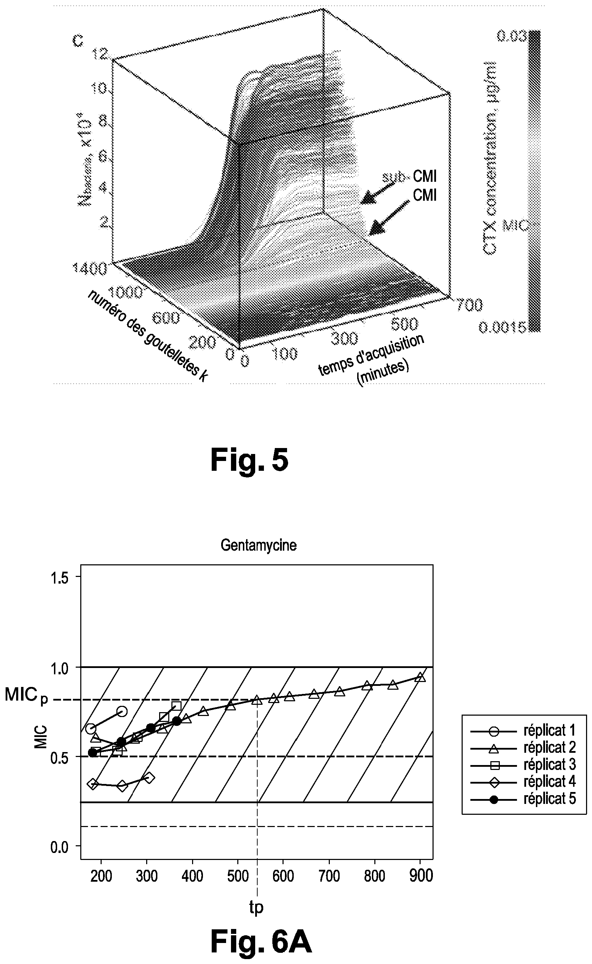

Les figures 4A et 4B illustrent par exemple les valeurs $x^k(t_p^k)$ en fonction du numéro $k$ des gouttelettes $k \in [1; N]$, respectivement pour le premier cycle de mesure et pour un cycle de mesure correspondant à $t_p$ = 400 minutes. Ces figures correspondent à la production de 1300 gouttelettes comprenant initialement chacune 1000 bactéries *E. coli* avec une concentration d'antibiotique « cefotaxime » (ou CTX) variant régulièrement de 0,0015µg.mL$^{-1}$ à 0.03µg.mL$^{-1}$, soit une précision sur la concentration inférieure à 10$^{-4}$ µg.mL$^{-1}$. On constate sur ces figures qu'une partie des gouttelettes voient leurs mesures $x^k(t_p^k)$ croitre par rapport à leurs instants initiaux respectifs $t_1^k$, ce qui signifie donc une croissance de la population des bactéries qu'elles contiennent, et corolairement que la concentration initiale d'antibiotique qu'elles contiennent était insuffisante pour totalement inhiber cette croissance. La figure 5 illustre le même phénomène en fonction de l'ensemble des instants de mesure compris entre 0 et 1400 minutes.

La détermination de la concentration minimale inhibitrice CMI est alors réalisée en incubant les gouttelettes pendant une durée jugée satisfaisante, puis en partageant les gouttelettes du dernier cycle de mesure $p$ en deux ensembles, à savoir entre celles dont le signal de mesure $x^k(t_P^k)$ reste sensiblement identique à leur signal initial $x^k(t_1^k)$ et celles dont le signal de mesure $x^k(t_P^k)$ est supérieur à leur signal initial $x^k(t_1^k)$. Il est ainsi déterminé le numéro $k_{ini}$ divisant les gouttelettes en ces deux ensembles. Le gradient de concentration étant défini en fonction du numéro k des gouttelettes, la concentration CMI est alors égale à la concentration initiale d'antibiotique de la gouttelette de numéro $k_{ini}$.

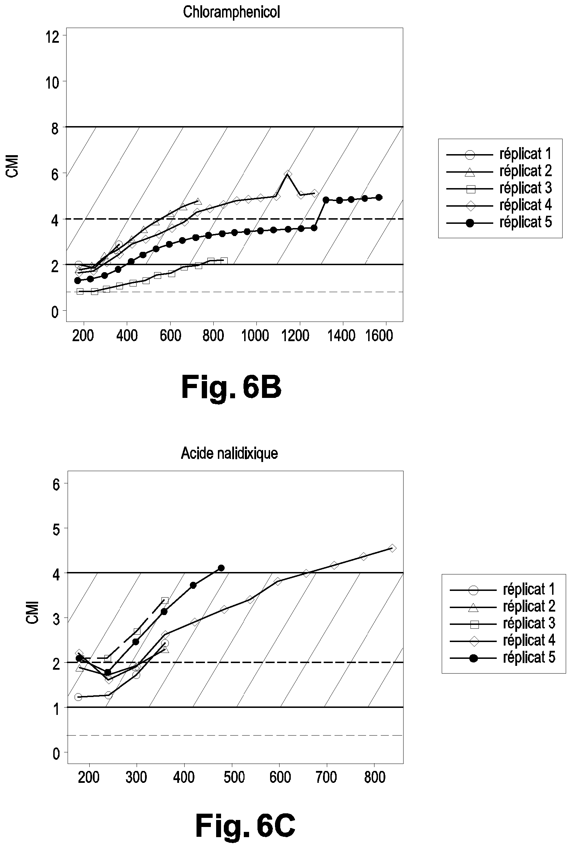

Les inventeurs ont mené des essais de détermination de concentrations CMI à l'aide du dispositif venant d'être décrit, ces essais étant illustrés aux figures 6A-6C. Ces dernières illustrent des essais menés sur 5 répliquats d'une souche de *E. Coli* (« répliquât 1 » à « répliquât 5 ») en présence de gentamycine (figure 6A), de chloramphénicol (figure 6B) ou d'acide nalidixique (figure 6C). Sur ces figures, les abscisses représentent le temps d'incubation des gouttelettes (en minutes) et les ordonnées la concentration MIC (en µg.mL$^{-1}$) déterminée par « découpage » de l'ensemble des gouttelettes tel que décrit ci-dessus. Un point d'une courbe, de coordonnées ($MIC_p, t_p$), représente donc la détermination de la concentration CMI en fonction des valeurs $x^k(t_p^k)$ des gouttelettes acquises pour le cycle de mesure $p$. La souche d'*E. Coli* utilisée pour les tests est une souche connue, de référence ATCC 25922, dont une CMI est connue pour les trois antibiotiques considéré. Le trait en pointillés de ces figures représente la concentration CMI reconnue par l'administration française, ou CMI « réglementaire » (0,5 µg.mL$^{-1}$ pour le gentamycine, 4 µg.mL$^{-1}$ pour le chloramphénicol et 2 µg.mL$^{-1}$ pour l'acide nalidixique) et la bande grisée autour de la CMI réglementaire correspond à l'intervalle de tolérance pour lequel une mesure de la CMI par un technique quelconque est jugée réglementairement conforme. La concentration CMI réglementaire est déterminée selon la technique manuelle décrite ci-dessus pour une durée d'incubation fixée réglementairement de 18 à 24h, et l'intervalle de tolérance correspond une dilution +1/-1 autour de cette concentration.

A la vue de ces résultats, on remarque notamment qu'il n'y a pas de convergence de la concentration CMI déterminée selon la méthode de « découpage », celle-ci continuant d'augmenter sur une longue période. Ce comportement de la concentration CMI pourrait s'expliquer en partie par le type de détection par fluorescence utilisée. En effet, dans le cadre d'un antibiotique bactériostatique, lorsque la fluorescence mesurée est celle de molécules rejetées par les bactéries suite à la digestion du milieu nutritif, la quantité de ces molécules peut augmenter alors que le nombre de bactérie reste sable. Toutefois, ceci n'expliquerait que très partiellement le comportement de la concentration MIC. Sans être lié à la théorie, les inventeurs pensent que les dispositifs tel que celui décrit précédemment mettent à jour des phénomènes qui étaient masqués par la technique de référence de détermination de la concentration CMI. Une détermination précise et robuste de la concentration minimale inhibitrice « réelle » (par opposition à la CMI « réglementaire ») en fonction des données produites par un dispositif aussi précis que celui décrit dans l'article « *Millifluidic droplet analyser for microbiology* », ou de tout autre dispositif analogue capable de produire un grand nombre de « incubateurs » en contrôlant avec précision leurs contenus initiaux, soulève donc de nouveau problème, et est donc en pratique difficile.

Ce même constat se pose également pour toute grandeur utilisée pour caractériser le pouvoir inhibiteur d'une, molécule sur des microorganismes (bactéries, levure, moisissure, etc), comme par exemple le taux de croissance des bactéries, la phase de latence de la croissance, le nombre maximum de bactéries par volume, des intervalles de concentration d'antibiotiques partiellement inhibitrices, etc.

Le procédé du document WO 2008/107881 consiste à déterminer lequel antibiotique, parmi un ensemble d'antibiotiques testés sur une bactérie, est efficace. Les documents US 2012/077206, WO 2014/155020, FR 2 916 761 décrivent des technologie d'identification d'antibiotiques effectifs sur une bactérie, d'incubation et de détermination de concentrations minimales inhibitrices.

**EXPOSE DE L'INVENTION**

Le but de la présente invention est de résoudre le problème susmentionné en proposant une détermination plus robuste et plus précise du pouvoir inhibiteur d'une molécule sur un microorganisme, par exemple la concentration MIC réelle.

A cet effet, l'invention a pour objet un procédé d'estimation d'une grandeur $G_{inhib}$ quantifiant le pouvoir inhibiteur d'une molécule sur un microorganisme d'un type prédéterminé, comprenant :

- la réalisation d'une pluralité d'échantillons, comprenant chacun au moins un microorganisme dudit type, un milieu nutritif pour le microorganisme et une quantité initiale de la molécule par microorganisme dudit type présent dans l'échantillon,
- ladite quantité initiale étant croissante dans un intervalle [$Q_{min}$, $Q_{max}$] en fonction d'un classement prédéterminé des échantillons ;
- l'incubation des échantillons ;
- pour chaque échantillon, la mesure de la croissance des microorganismes dans l'échantillon en fonction du temps pendant une durée d'incubation prédéterminée ; et
- la détermination de la grandeur $G_{inhib}$ en fonction des mesures de la croissance des microorganismes dans les échantillons.

Selon l'invention, la détermination de la grandeur $G_{inhib}$ comprend :

- pour chaque échantillon, le calcul d'une valeur reflétant la croissance du microorganisme dudit type en fonction de la mesure de croissance des microorganismes dans l'échantillon;
- le classement des valeurs calculées pour les échantillons en fonction du classement des échantillons ; et
- la détermination de la grandeur $G_{inhib}$ en fonction de l'évolution des valeurs classées.

En d'autres termes, la détermination de la grandeur $G_{inhib}$ n'est pas réalisée sur les valeurs $x^k(t_P^k)$, ou toutes autres grandeurs qui y seraient directement liées, comme par exemple la taille de la population bactérienne calculée en fonction de ces valeurs pour un temps $t_P$ ou un cycle $P$ donné. Une information sur la dynamique de croissance des bactéries est tout d'abord déterminée en fonction des valeurs $\{x^k(t_1^k), x^k(t_2^k), ..., x^k(t_p^k), ..., x^k(t_P^k)\}$ et c'est cette information qui est traitée ensuite pour déterminer la grandeur $G_{inhib}$.

Cette information est recherchée avantageusement en se fondant sur une connaissance a priori du comportement des bactéries, notamment à l'aide d'un modèle de croissance dont on identifie au moins l'un des paramètres contenant une information sur la dynamique de croissance. Comme cela sera détaillé ci-après, la détermination de la grandeur $G_{inhib}$ se fait alors rapidement, de manière plus précise et reproductible. En outre, l'ensemble du procédé est automatisé et donc moins dépendant de l'interprétation des opérateurs.

Selon un mode de réalisation :

- la croissance du microorganisme est modélisée par un modèle de croissance en fonction du temps comprenant :

  - une première phase de latence de durée $\lambda$ ;
  - suivie d'une seconde phase de croissance exponentielle de pente maximale $\mu$ dans l'espace logarithmique ; et
  - suivie d'une troisième phase stationnaire de valeur maximale A ;

- et la valeur reflétant la croissance du microorganisme dudit type en fonction du temps est une estimation de la pente maximale $\mu$ et/ou une estimation de la durée de la phase de latence $\lambda$.

En d'autres termes, le profil de croissance correspond à celui proposé par Rosso L. dans sa thèse « Modélisation et microbiologie prévisionnelle : élaboration d'un nouvel outil pour l'agroalimentaire. » Thèse de doctorat, Université Claude Bernard Lyon 1, 1995. La pente $\mu$ et la phase de latence $\lambda$ sont en effet chacune directement liée à la dynamique de croissance des bactéries.

Selon un mode de réalisation :

- la grandeur $G_{inhib}$ comprend un intervalle $[Q_{min}^{MIC}, Q_{max}^{MIC}]$ pour lequel la croissance des microorganismes dans les échantillons est au moins partiellement inhibée ;

- la quantité initiale de la molécule en fonction du classement des échantillons comprend :

  ▪ une première partie constante sur plusieurs échantillons et égale à $Q_{min}$, la borne inférieure $Q_{min}$ de l'intervalle $[Q_{min}, Q_{max}]$ étant choisie de sorte qu'il n'y a aucune inhibition de la croissance des microorganismes dans les échantillons ;
  ▪ suivie d'une seconde partie strictement croissante de $Q_{min}$ à $Q_{max}$ ;
  ▪ suivie d'une troisième partie constante sur plusieurs échantillons et égale à $Q_{max}$, la borne supérieure $Q_{max}$ de l'intervalle $[Q_{min}, Q_{max}]$ étant choisie de sorte qu'il y a une inhibition totale de la croissance des microorganismes dans les échantillons ;

- et la détermination de l'intervalle $\left[Q_{min}^{MIC}, Q_{max}^{MIC}\right]$ comprend :

  ▪ l'identification d'une zone de transition dans l'évolution des valeurs classées entre deux zones extrêmes sensiblement stationnaires de ladite évolution; et
  ▪ la détermination de l'intervalle $\left[Q_{min}^{MIC}, Q_{max}^{MIC}\right]$ comme étant l'intervalle correspondant aux échantillons de la zone de transition identifiée.

En d'autres termes, grâce à la construction particulière des échantillons, il est possible d'identifier de manière plus aisée l'intervalle $\left[Q_{min}^{MIC}, Q_{max}^{MIC}\right]$ dans lequel. Cet intervalle, qui est la zone de transition de l'effet inhibiteur de la molécule, entre aucun effet inhibiteur et un effet inhibiteur complet, est en soi une grandeur utile $G_{inhib}$ et comporte en outre d'autres types d'informations utiles, comme par exemple la concentration MIC.

Notamment, l'identification de la zone de transition comprend la détermination de deux points d'inflexion de l'évolution des valeurs classées, la zone de transition étant bornée par les deux points d'inflexion déterminés. Notamment, l'indentification de la zone de transition comprend la modélisation de l'évolution des valeurs classées par une fonction continue linéaire par morceau comprenant uniquement deux segments de droite extrêmes et un segment de droite intermédiaire compris entre les deux segments de droite extrême, le segment de droite intermédiaire étant la zone de transition.

Selon une variante avantageuse, la grandeur $G_{inhib}$ comprend une quantité initiale minimale de molécules $Q_{MIC}$ qui est totalement inhibitrice de la croissance des microorganismes, et ladite quantité initiale minimale inhibitrice $Q_{MIC}$ étant choisie égale à la borne supérieure $Q_{max}^{MIC}$ de l'intervalle $\left[Q_{min}^{MIC}, Q_{max}^{MIC}\right]$.

Selon un mode de réalisation, la borne inférieure $Q_{min}$ de l'intervalle $[Q_{min}, Q_{max}]$ est une quantité nulle de la molécule, par exemple un antibiotique.

Selon un mode de réalisation :

- les mesures de la croissance des bactéries dans les échantillons et la détermination de la grandeur $G_{inhib}$ en fonction desdites mesures sont mises en œuvre pour des durées d'incubation croissantes de manière à obtenir une séquence de grandeur $G_{inhib}$ en fonction de la durée d'incubation des échantillons ;
- le procédé comprend l'analyse de la stabilité de ladite séquence en fonction de la durée d'incubation; et
- la grandeur $G_{inhib}$ est la valeur de la séquence une fois la séquence stabilisée.

En d'autres termes, l'invention permet de déterminer une séquence correspondant à la grandeur $G_{inhib}$ qui est convergente, ce qui permet de mettre en œuvre un test de stabilité, et donc un test permettant la détection et l'arrêt automatique de l'incubation et/ou du traitement des données. Notamment, la précision de l'estimation augmente avec la longueur de la séquence.

Selon un mode de réalisation, les échantillons comprennent chacun initialement au moins 100 microorganismes, et de préférence au moins 500 microorganismes. En d'autres termes, prévoir un nombre initial minimal de bactéries évite d'exacerber la particularité d'une bactérie particulière. Bien entendu, grâce à l'invention, il est également possible d'étudier une population plus réduite, voir même un microorganisme si l'on souhaite connaître l'effet de la molécule sur ce microorganisme en particulier, comme pour l'étude des phénomènes d'hétéro-résistance d'une bactérie par exemple.

Selon un mode de réalisation, les échantillons comprennent chacun une quantité initiale d'une deuxième molécule différente apte à inhiber la croissance des microorganismes, notamment une quantité initiale identique pour tous les échantillons. En d'autres termes, l'invention permet d'étudier les effets de synergie entre des agents inhibiteurs, par exemple des antibiotiques.

Selon un mode de réalisation, la quantité minimale de la molécule par microorganisme dudit type est une concentration de la molécule dans les échantillons, la concentration initiale de microorganisme dudit type dans les échantillons étant constante en fonction du classement des échantillons.

Selon un mode de réalisation, le microorganisme est une bactérie, et la molécule est un antibiotique. En variante, le microorganisme est une levure ou une moisissure, et la molécule est un antifongique.

Selon un mode de réalisation, le milieu nutritif comporte un élément métabolisable par le microorganisme sous la forme de molécule fluorescente, et la mesure de la croissance des microorganismes dans les échantillons est une mesure de la fluorescence des échantillons. En variante, l'absorbance des échantillons est variable en fonction de la quantité de microorganismes présents dans ceux-ci, et en ce que la mesure de la croissance des microorganismes dans les échantillons est une mesure de densité optique.

Selon un mode de réalisation, la réalisation de la pluralité d'échantillon comporte la réalisation d'un train de gouttes formant échantillons dans de l'huile.

L'invention a également pour objet un dispositif d'estimation d'une grandeur $G_{inhib}$ quantifiant le pouvoir inhibiteur d'une molécule sur un microorganisme d'un type prédéterminé, comprenant :

- des moyens pour la réalisation d'une pluralité d'échantillons, comprenant chacun au moins un microorganisme dudit type, un milieu nutritif pour le microorganisme et une quantité initiale de la molécule par microorganisme dudit type présent dans l'échantillon, ladite quantité initiale étant croissante dans un intervalle [$Q_{min}$, $Q_{max}$] en fonction d'un classement prédéterminé des échantillons ;
- des moyens pour l'incubation des échantillons ;
- des moyens pour la mesure de la croissance des microorganismes dans chaque échantillon en fonction du temps pendant une durée d'incubation prédéterminée ; et
- des moyens de calcul configurés pour la détermination de la grandeur $G_{inhib}$ en fonction des mesures de la croissance des microorganismes dans les échantillons.

Selon l'invention, les moyens de calcul sont configurés pour mettre en œuvre :

- pour chaque échantillon, le calcul d'une valeur d'un paramètre d'un modèle paramétrique de croissance du microorganisme dudit type en fonction de la mesure de croissance des microorganismes dans l'échantillon;
- le classement des valeurs calculées pour les échantillons en fonction du classement des échantillons ; et
- la détermination de la grandeur $G_{inhib}$ en fonction de l'évolution des valeurs classées.

Notamment, le dispositif est configuré pour mettre en oeuvre un procédé du type précité.

**BREVE DESCRIPTION DES FIGURES**

L'invention sera mieux comprise à la lecture de la description qui suit, à l'appui des figures annexées, dans lesquelles :

- la figure 1 est une vue schématique simplifiée de l'analyseur décrit dans l'article « *Millifluidic droplet analyser for microbiology* » ;
- la figure 2 est un exemple de gouttelettes produites par l'analyseur de la figure 1 ;
- la figure 3 est un schéma décrivant la production de signaux de fluorescence par l'analyseur de la figure 1 ;
- les figures 4A et 4B sont des tracés de mesures de fluorescence en fonction du numéro des gouttelettes produites, respectivement à 0 minute et 400 minutes ;
- la figure 5 est un tracé de mesures de fluorescence en fonction du numéro des gouttelettes produites et du temps ;
- les figures 6A à 6C sont des tracés illustrant la détermination de la concentration minimale inhibitrice selon une méthode de découpage de l'état de la technique, pour des essais réalisés sur l'*E. Coli* avec trois antibiotiques différents ;
- la figure 7 est un organigramme d'un mode de réalisation du procédé selon l'invention ;
- la figure 8 est un tracé illustrant un profil de concentration initiale d'antibiotique dans les gouttelettes généré lors du procédé selon l'invention ;
- la figure 9 est un tracé illustrant des consignes de débit des seringues de l'analyseur de la figure 1, généré en fonction du profil de la figure 8 ;
- la figure 10 est un tracé des mesures de fluorescence des gouttelettes produites par les consignes de la figure 9 en fonction du temps ;
- la figure 11 est un tracé illustrant des mesures de fluorescences en fonction du numéro des gouttelettes pour différents instants de mesure ;
- les figures 12A et 12B sont des tracés illustrant l'estimation de la concentration initiale réelle d'antibiotique dans les gouttelettes pour deux essais différents ;
- la figure 13 est un tracé illustrant la croissance de bactérie en présence de nutriments et en fonction du temps ;
- la figure 14 est un tracé illustrant la transformation des mesures de fluorescence en séquence de taux maximum de croissance des bactéries en fonction du numéro des gouttelettes ;
- la figure 15 est un tracé illustrant la transformation des mesures de fluorescence en séquences de durées de phase de latence de la croissance des bactéries en fonction du numéro des gouttelettes ;
- les figures 16A et 16B sont des tracés illustrant respectivement une phase de transition dans une séquence de taux maximum de croissance et l'approximation de la séquence de taux maximum de croissance par une fonction linéaire par morceaux ;
- les figures 17 et 18 sont des tracés illustrant des zones de transition obtenues respectivement sur une séquence de taux maximum de croissance et une séquence de durées de phase de latence ; et
- les figures 19A à 19C sont des tracés illustrant la détermination de la concentration minimale inhibitrice selon l'invention, pour les essais réalisés sur l'*E. Coli* avec trois antibiotiques différents des figures 6A à 6C.

## DESCRIPTION DETAILLEE DE L'INVENTION

### EXEMPLE DE REALISATION

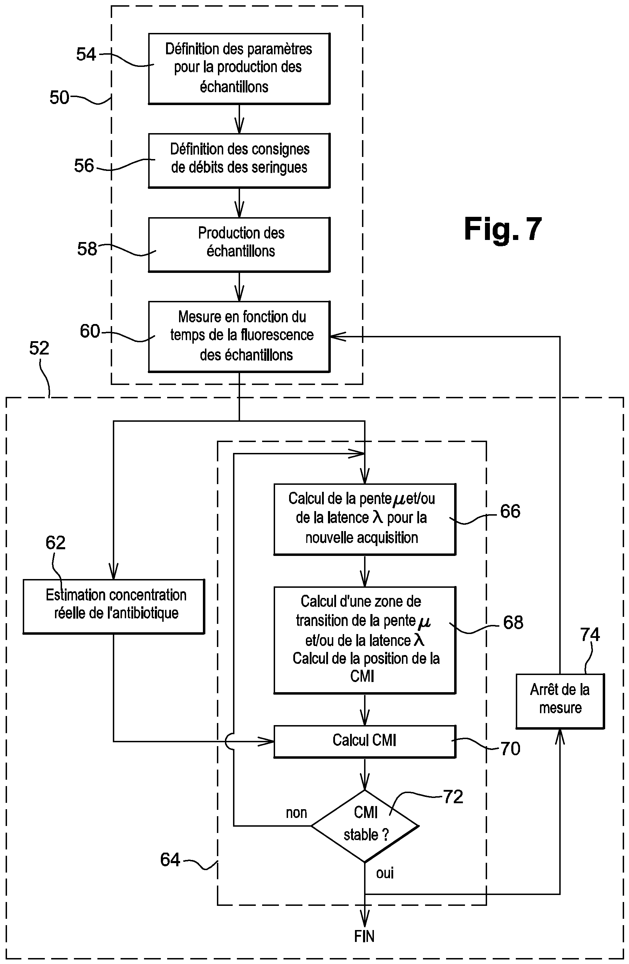

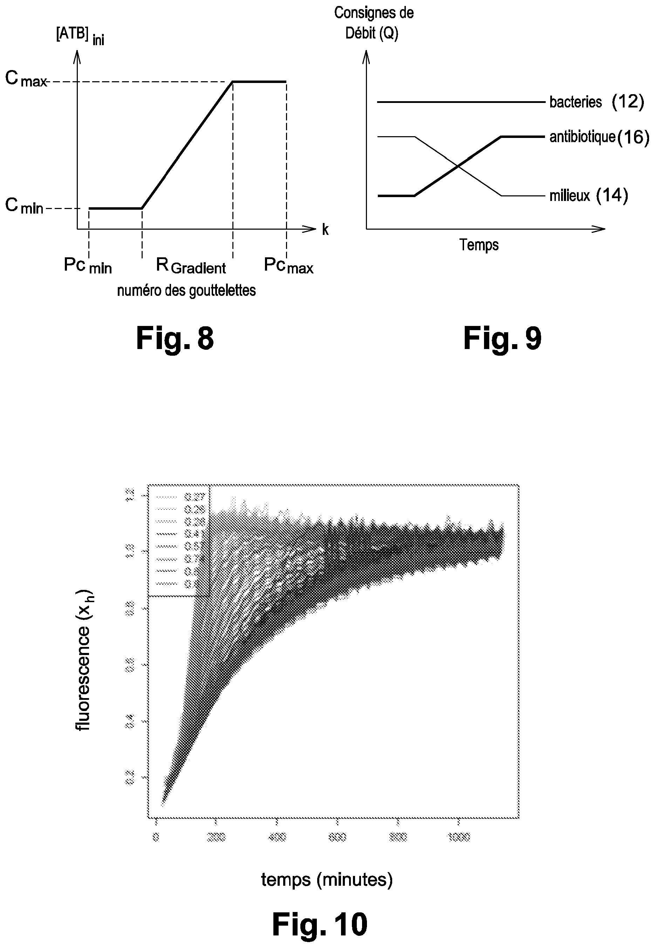

Il va à présent être décrit en relation avec l'organigramme de la figure 7 un mode de réalisation du procédé selon l'invention, des étapes de ce procédé étant illustrées par les figures 8 à 19. Le procédé est appliqué à la détermination d'une concentration minimale inhibitrice MIC de la croissance de bactéries, à l'aide du dispositif **10** décrit dans l'article *« Millifluidic droplet analyser for microbiology »* et sommairement décrit ci-dessus en relation avec la figure 1. La commande des organes de ce dispositif et le traitement des mesures sont réalisés à l'aide d'une unité de traitement d'information classique, par exemple un ordinateur.

Le procédé comporte la production, en **50,** de données expérimentales sur la croissance de bactéries en présence d'un gradient d'antibiotique, et l'analyse, en **52,** des données produites pour déterminer la concentration MIC.

L'étape de production **50** comporte une première étape **54** de détermination de paramètres pour la production des données. L'étape **54** comprend notamment la définition d'un intervalle de concentration $[C_{min}; C_{max}]$ dans lequel la concentration CMI est supposément comprise, à savoir $C_{min} < CMI < C_{max}$. Cet intervalle est déterminé en fonction d'études précédentes, notamment en fonction d'une concentration CMI réglementaire ou d'études cliniques. Notamment, la concentration $C_{max}$ est une concentration pour laquelle l'antibiotique inhibe totalement la croissance bactérienne et supérieure à la concentration MIC. En variante, le procédé décrit ci-dessous sert à régler l'intervalle $[C_{min}; C_{max}]$. Par exemple, si la concentration CMI déterminée est très éloignée de la concentration maximale $C_{max}$, cette dernière est diminuée et le procédé mis en œuvre une fois de plus. De même, si la concentration MIC est trop proche de la concentration maximale $C_{max}$, cette dernière est augmentée et le procédé relancé. De préférence, la concentration minimale $C_{min}$ est choisie de manière à garantir que les bactéries soient sensiblement libres de croître, une telle croissance libre étant ultérieurement exploitée lors du traitement des données, comme cela sera expliqué plus en détail ci-dessous. Par exemple, la concentration $C_{min}$ est égale à 0.

Un profil de concentration initiale d'antibiotique $[ATB]_{ini}$ en fonction du numéro $k$ des gouttelettes ultérieurement produites est alors généré tel qu'illustré à la figure 8. Ce profil comprend :

- un premier plateau $P_{C_{min}}$ pour lequel $\forall k \in [1; N_{C_{min}}]$, $[ATB]_{ini}(k) = C_{min}$ ;
- suivi d'une rampe $R_{gradient}$ pour laquelle la concentration $[ATB]_{ini}(k)$ augmente linéairement depuis la concentration minimale $C_{min}$ jusqu'à la concentration maximale $C_{max}$, c'est-à-dire $\forall k \in [N_{C_{min}} + 1; N_{gradient}]$, $[ATB]_{ini}(k + 1) - [ATB]_{ini}(k) = constante$;
- suivi d'un second plateau $P_{C_{max}}$ pour lequel $\forall k \in [N_{gractient}; N]$, $[ATB]_{ini}(k) = C_{max}$.

Les longueurs des plateaux $P_{C_{min}}$ et $P_{C_{max}}$ sont choisies de manière à identifier automatiquement des portions de droites de pente sensiblement nulles en fonction du numéro $k$ dans les données produites ultérieurement. Ces longueurs dépendent par exemple de la précision de l'algorithme utilisé. Les inventeurs ont cependant noté qu'une longueur de plateaux égale à une centaine de gouttelettes permet une identification de bonne qualité. La longueur de la rampe $R_{gradient}$ est quant à elle définie en fonction de la précision souhaitée concernant la concentration MIC, dans les limites imposées par le dispositif de production des gouttelettes.

Des consignes de débit pour les seringues **12, 14, 16** sont ensuite produites, en **56,** en fonction du profil de concentration initiale d'antibiotique $[ATB]_{ini}$. Ces consignes sont illustrées à la figure 9. Notamment, la consigne de débit de la seringue **12** de solution bactérienne est constante afin de produire des gouttelettes comportant sensiblement le même nombre initial de bactéries. Ce nombre est avantageusement supérieur à 500 pour ne pas exacerber les particularités de chaque bactérie, par exemple 1000 bactéries. La consigne de débit de la seringue **16** d'antibiotique suit quant à elle le profil [ATB]$_{ini}$ et la consigne de débit de la seringue **18** de milieu nutritif présente un profil inversé afin de produire des gouttelettes de volume constant.

En parallèle, les solutions bactériennes, de milieu nutritif et d'antibiotique sont préparées puis mises dans leurs seringues respectives. De manière avantageuse, et optionnelle, un marqueur fluorescent, par exemple de la sulforhodamine, de concentration connue, est également ajouté à la solution antibiotique. Ce marqueur, dont la fluorescence est mesurable par le système de détection **28**, avantageusement à une longueur d'onde différente de celle utilisée pour mesurer la population des bactéries, permet de déterminer la concentration réelle d'antibiotique dans chaque gouttelette, comme cela sera expliqué en détail ci-dessous. La mesure de cette fluorescence supplémentaire est réalisée par le système de détection **38** qui est équipé par exemple d'un jeu de filtre qui permet de sélectionner la longueur d'onde mesurée, telle que cela est par exemple décrit dans le document « *Millifluidic droplet analyser for microbiology* ».

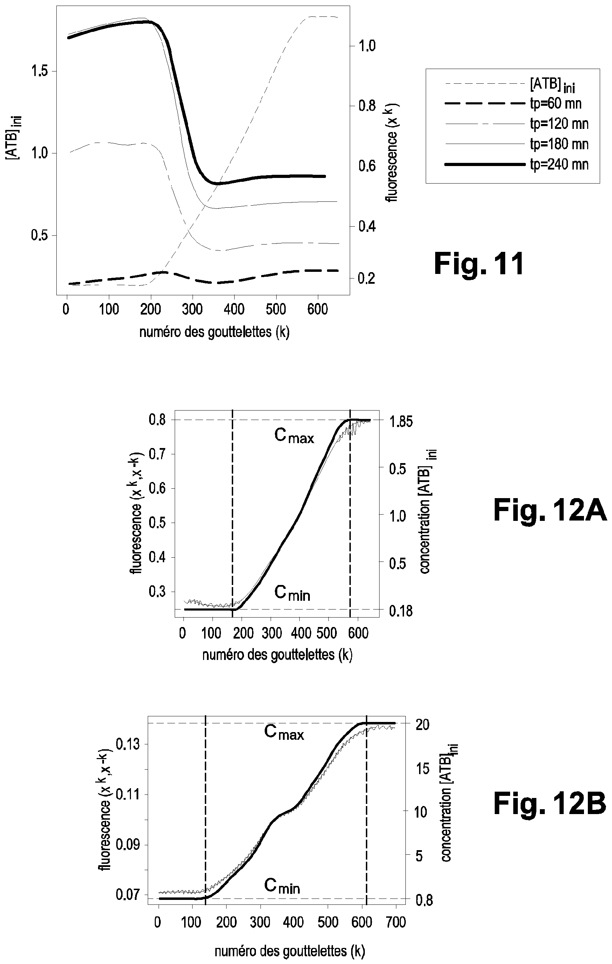

Dans une étape **60** suivante, le dispositif **10** est piloté en fonction des consignes de débit ainsi définies afin de produire un train de *N* gouttelettes, et la fluorescence de chaque gouttelette est mesurée régulièrement en mettant en œuvre le mouvement de va-et-vient décrit précédemment. Toujours en **60,** le signal de mesure issu du système de détection **28** est traité pour produire et mémoriser les valeurs de fluorescence $\left\{ x^k(t_1^k), x^k(t_2^k), \ldots, x^k(t_p^k), \ldots, x^k(t_P^k) \right\}$ de chaque gouttelette pour les instants d'acquisition $\left\{ t_1^k, t_2^k, \ldots, t_p^k, \ldots, t_P^k \right\}$. Un exemple de grandeurs $x^k(t_p^k)$ est illustré aux figures 10 et 11, soit en fonction du temps $t_p^k$ (figure 10) soit en fonction du numéro des gouttelettes pour différents cycles de mesure (figure 11).

L'étape **52** de traitement des données comprend quant à elle l'estimation, en **62,** de la concentration initiale réelle d'antibiotique dans les gouttelettes. En pratique, il y a une différence entre les consignes de débit et les débits réels de sorte qu'il y a une différence entre le profil [ATB]$_{ini}$ souhaité et le profil réel de la concentration. Notamment, le profil réel peut ne pas être parfaitement linéaire. L'estimation de la concentration réelle d'antibiotique est réalisée sur la fluorescence de la sulforhodamine mesurée $\left\{ z^1(t_L^1), z^2(t_L^2), \ldots, z^k(t_L^k), \ldots, z^N(t_L^N) \right\}$ au début de l'incubation des gouttelettes. Le cycle de mesure *L* est notamment compris dans la phase de latence des bactéries, et est par exemple le premier cycle de mesure. A cet instant, les bactéries n'ont pas commencé à croitre et induisent une fluorescence constante ou nulle dans les gouttelettes. La variation de la fluorescence parmi les valeurs $\left\{ z^1(t_L^1), z^2(t_L^2), \ldots, z^k(t_L^k), \ldots, z^N(t_L^N) \right\}$ correspond donc à la fluorescence de la sulforodhamine ajoutée à la solution d'antibiotique. Connaissant la concentration de la sulforodhamine, la fluorescence de cette dernière est donc proportionnelle à la concentration initiale de l'antibiotique [ATB]$_{ini}$.

L'estimation $[\widehat{ATB}]_{ini}$ de la concentration réelle est calculée notamment en :

- appliquant un filtre de lissage sur les mesures $\left\{ z^1(t_L^1), z^2(t_L^2), \ldots, z^k(t_L^k), \ldots, z^N(t_L^N) \right\}$, par exemple un filtre de lissage de Loess standard, de manière à obtenir des mesures lissées $\{\bar{z}^1, \bar{z}^2, \ldots, \bar{z}^k, \ldots, \bar{z}^N\}$;
- identifiant le début et la fin du gradient d'antibiotique dans les mesures lissées. Par exemple, la valeur minimale $z^{Nmin} = min\{\bar{z}^1, \bar{z}^2, \ldots, \bar{z}^k, \ldots, \bar{z}^N\}$ des mesures lissées est identifiée et le début du gradient est identifié comme le plus petit numéro $N_g^{min} > N_{min}$ de la gouttelette dont la mesure lissée $\bar{z}^{N_g^{min}}$ est supérieure de X% à la valeur $z^{Nmin}$, par exemple de 1%. De manière analogue, la valeur maximale $z^{Nmax} = max\{\bar{z}^1, \bar{z}^2, \ldots, \bar{z}^k, \ldots, \bar{z}^N\}$ des mesures lissées est identifiée et la fin du gradient est identifiée comme le plus grand numéro $N_g^{max} < N_{max}$ de la gouttelette dont la mesure lissée $\bar{z}^{N_g^{max}}$ est inférieure de X% à la valeur $z^{Nmax}$, par exemple 99%. Bien entendu toute méthode pour déterminer le début et la fin du gradient peut être employée ;
- en posant :

$$[\widehat{ATB}]_{ini}(k) = \begin{cases} C_{min} & \forall k \in \left[1 ; N_g^{min}\right[ \\ a.\bar{z}^k + b & \forall k \in \left[N_g^{min} ; N_g^{max}\right] \\ C_{max} & \forall k \in \left[N_g^{max} + 1 ; N\right] \end{cases} \qquad (1)$$

avec $a = \frac{C_{max} - C_{min}}{\bar{z}^{N_g^{max}} - \bar{z}^{N_g^{min}}}$ et $b = \frac{C_{max} + C_{min}}{2} - a \times \frac{\bar{z}^{N_g^{max}} + \bar{z}^{N_g^{min}}}{2}$. La concentration estimée $\widehat{[ATB]}_{ini}(k)$ est mémorisée pour une utilisation ultérieure telle que décrite ci-dessus.

Les concentrations connues $C_{min}$ et $C_{max}$ servent ainsi de point d'ancrage à la transformation linéaire du gradient de fluorescence compris dans la plage $\left[\bar{z}^{N_g^{min}}; \bar{z}^{N_g^{max}}\right]$ en un gradient de concentration $\widehat{[ATB]}_{ini}$ dans la gamme $[C_{min}; C_{max}]$. Notamment, ceci permet de conserver les non linéarités du profil réel de concentration initiale induit par les erreurs dans la production des gouttelettes. Les figures 12A et 12B illustrent l'estimation du profil de concentration $[ATB]_{ini}$ pour deux expériences menées respectivement pour deux souches d'E.Coli. Les courbes bruitées représentent la fluorescence mesurée $\left\{z^1(t_L^1), z^2(t_L^2), \dots, z^k(t_L^k), \dots, z^N(t_L^N)\right\}$, les courbes lissées (en gras) se superposant aux courbes bruitées correspondent à la fluorescence lissée $\{\bar{z}^1, \bar{z}^2, \dots, \bar{z}^k, \dots, \bar{z}^N\}$, et les courbes ancrées sur les valeurs $C_{min}$ et $C_{max}$ (en trait fin) sont la concentration estimée $\widehat{[ATB]}_{ini}$. On remarque sur ces figures, et particulièrement sur la figure 12B, l'importante non linéarité de la fluorescence mesurée, provoquée par les imperfections du dispositif **10**, et l'estimation $\widehat{[ATB]}_{ini}$ de la concentration qui reproduit, à un facteur d'échelle près, le profil de la fluorescence.

Le traitement **52** comporte également une étape **64** mise en œuvre en parallèle de l'étape de mesure **60**, à savoir à chaque fois qu'un nouveau cycle de mesure $P$ délivre de nouvelles mesures $\left\{x^1(t_P^1), x^2(t_P^2), \dots, x^k(t_P^k), \dots, x^N(t_P^N)\right\}$ de la fluorescence des gouttelettes, et ceci tant qu'un critère d'arrêt décrit ci-après n'est pas satisfait. Lors du déclenchement de l'étape **64**, des mesures $\left\{x^k(t_1^k), x^k(t_2^k), \dots, x^k(t_p^k), \dots, x^k(t_{P-1}^k)\right\}$, correspondant aux cycles de mesure précédents 1, 2, ..., $P$-1, ont donc déjà été mémorisées pour chaque gouttelette $k$.

Plus particulièrement, pour chaque gouttelette $k$, l'étape 64 comporte une première étape **66** de transformation de la séquence $\left\{x^k(t_1^k), x^k(t_2^k), \dots, x^k(t_p^k), \dots, x^k(t_P^k)\right\}$, provenant de la concaténation de la séquence mémorisée $\left\{x^k(t_1^k), x^k(t_2^k), \dots, x^k(t_p^k), \dots, x^k(t_{P-1}^k)\right\}$ avec la nouvelle mesure de fluorescence $x^k(t_P^k)$ de la gouttelette, en une valeur $D^k(t_P)$ contenant une information sur la dynamique de croissance des bactéries dans la gouttelette $k$ pendant une période d'incubation comprise entre $t_1$ et $t_P$. L'objectif de cette transformation est de tenir compte, pour le cycle de mesure d'instant $t_P$, de l'historique de la fluorescence jusqu'à la réalisation de ce cycle, tout en qualifiant de manière qualitative cet historique, avantageusement au travers d'un modèle de croissance.

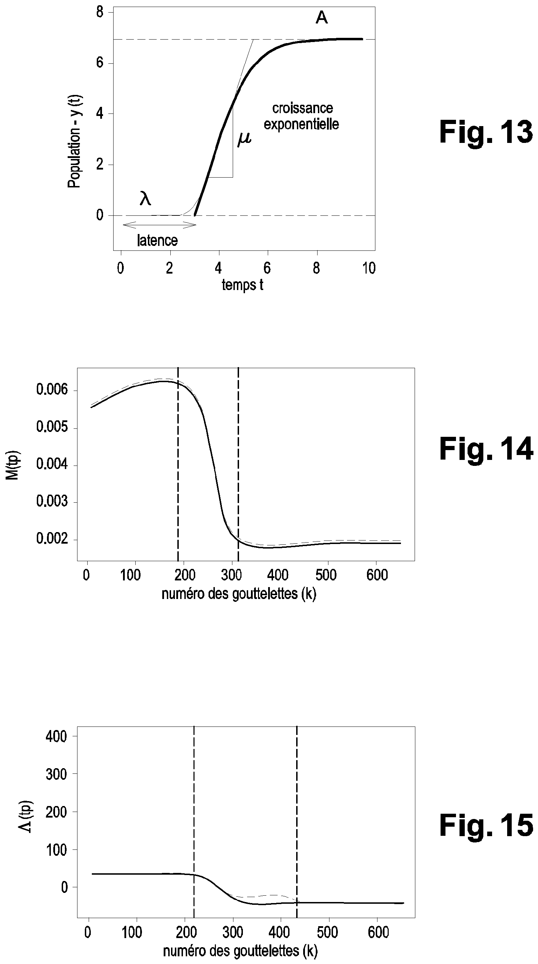

Cet historique est avantageusement pris en compte au travers d'un modèle de la croissance de bactéries dans un milieu nutritif, plus préférentiellement le modèle de la figure 13 qui illustre le logarithme népérien de la population bactérienne en fonction du temps. Comme cela est connu, la croissance des bactéries comporte :

- une première phase de latence de durée $\lambda$ pendant laquelle les bactéries synthétisent des enzymes qui vont leur être nécessaire pour utiliser le milieu nutritif et sans division cellulaires des bactéries ;
- suivie d'une phase de croissance exponentielle: après une accélération, la croissance atteint un taux de croissance maximum $\mu$, ou de manière équivalente la courbe de croissance présente une pente maximale $\mu$ ;
- suivi d'une phase stationnaire qui correspond à l'épuisement du milieu nutritif. La croissance décélère et devient sensiblement nulle, la population de bactérie étant sensiblement stabilisée à une valeur $A$. La phase stationnaire est suivie par une phase de déclin, non représentée ici, faisant suite à l'épuisement total des nutriments.

Les phases de latence, de croissance et stationnaires sont estimées par exemple par l'une et/ou l'autre des modèles temporels $y(t)$ du tableau suivant :

| Nom du modèle | Formule $y(t)$ | Paramètres à identifier |
|---|---|---|
| Logistique | $$y(t) = \frac{A}{1 + exp\left(\frac{4.\mu}{A}.(\lambda - t) + 2\right)}$$ | A, $\mu$, $\lambda$ |
| Gompertz | $$y(t) = A.exp\left(-exp\left(\frac{\mu.e}{A}.(\lambda - t)\right) + 1\right)$$ | A, $\mu$, $\lambda$ |
| Gompertz modifié | $$y(t) = A.exp\left(-exp\left(\frac{\mu.e}{A}.(\lambda - t)\right) + 1\right) + A.exp\left(\alpha.(t - t_{shift})\right)$$ | A, $\mu$, $\lambda$, $\alpha$, $t_{shift}$ |
| Richards | $$y(t) = A.\left(1 + v.exp\left(1 + v + \frac{\mu}{A}.(1 + v)^{1+\frac{1}{v}}\right).(\lambda - t)\right)^{\left(-\frac{1}{v}\right)}$$ | A, $\mu$, $\lambda$, $v$ |

où $e$ est la constante d'Euler.

Pour chaque cycle de mesure $P$ et pour chaque gouttelette $k$, l'étape **66** consiste ainsi à identifier au moins un des paramètres d'un modèle $y(t)$ contenant l'information de dynamique en fonction des fluorescences mesurées $\left\{x^k(t_1^k), x^k(t_2^k), \ldots, x^k(t_p^k), \ldots, x^k(t_P^k)\right\}$ pour la gouttelette, et notamment une pente maximale $\mu^k(t_P)$ et/ou une durée de latence $\lambda^k(t_p)$ pour cette séquence ($D^k(t_P) = \mu^k(t_P)$ ou $D^k(t_P) = \lambda^k(t_P)$). L'identification des paramètres du modèle $y(t)$, qui consiste à minimiser une erreur d'estimation formée de la différence entre le vecteur des mesures $\left(x^k(t_1^k) \quad x^k(t_2^k) \quad \ldots \quad x^k(t_p^k) \quad \ldots \quad x^k(t_P^k)\right)^T$ et le vecteur d'estimation des mesures $\left(y(t_1^k) \quad y(t_2^k) \quad \ldots \quad y(t_p^k) \quad \ldots \quad y(t_P^k)\right)^T$, est réalisée de manière connue en soi du domaine de l'identification, par exemple par un moindre carrée non linéaire. En variante, les paramètres sont identifiés sans utilisation de modèle $y(t)$, par exemple en calculant un polynôme par la méthode des splines approximant la séquence $\left(x^k(t_1^k) \quad x^k(t_2^k) \quad \ldots \quad x^k(t_p^k) \quad \ldots \quad x^k(t_P^k)\right)^T$. Les paramètres $\lambda$ et $\mu$ sont ensuite estimés de manière empirique, par exemple par la méthode des différences finies. Par exemple, la pente maximale $\mu$ est obtenue en calculant la dérivée du polynôme approximant la séquence et en sélectionnant la valeur maximale de la dérivée en tant que pente $\mu$. En variante encore, les modèles ou les approches peuvent être mixées.

L'identification des paramètres de la croissance d'une population bactérienne est bien connue de l'état de la technique. Par exemple, cette identification est réalisée à l'aide du paquet logiciel (« package ») « grofit » décrit dans le document de Kahm M. et al. « grofit : Fitting Biological Growthe Curve with R », Journal of Statistical Software, vol. 33(7), février 2010, téléchargeable à l'URL http://cran.r-project.org/web/packages/grofit/index.html.

Le calcul des paramètres étant de nature statistique, l'identification est de préférence réalisée une fois qu'un nombre minimal de mesures a été acquis. Le nombre minimum de cycles de mesure est par exemple égal 10, l'étape **64** étant donc mise en œuvre pour cycles de mesure une fois ce nombre minimum atteint.

A l'issue de l'étape **66** de calcul des paramètres de la croissance des bactéries, il est donc produit, les séquences :

$$M(t_P) = \{\mu^1(t_P), \mu^2(t_P), \ldots, \mu^k(t_P), \ldots, \mu^N(t_P)\}$$

$$\Lambda(t_P) = \{\lambda^1(t_P), \lambda^2(t_P), \ldots, \lambda^k(t_P), \ldots, \lambda^N(t_P)\}$$

Une séquence M($t_P$) et une séquence A($t_P$) sont illustrées respectivement aux figures 14 et 15 en fonction du numéro $k$ des gouttelettes, pour un instant $t_P$ égal à 6 heures.

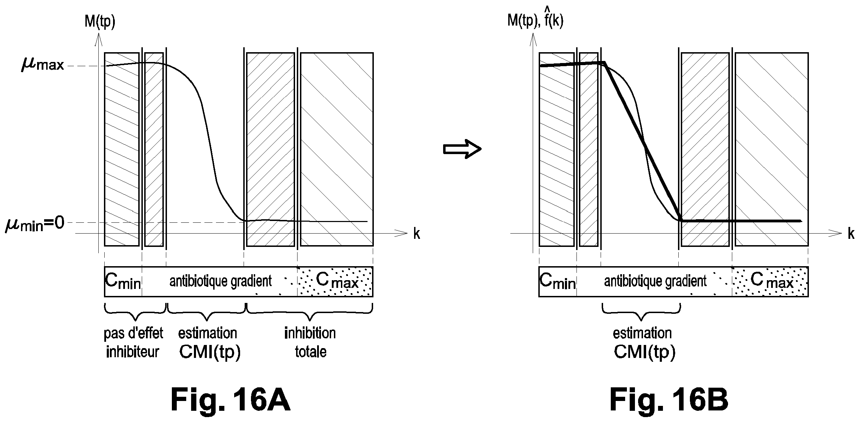

Le traitement **52** se poursuit, en **68,** par la détermination d'une concentration minimale inhibitrice réelle $CMI(t_P)$ pour l'instant $t_P$ en fonction d'au moins l'une des séquences de paramètres déterminées, par exemple la séquence $M(t_P)$. Cette détermination se fonde sur la recherche d'une zone de transition dans la séquence de paramètres comportant la concentration $CMI(t_P)$. Cette zone se définit comme la gamme de concentrations initiales d'antibiotique de largeur minimale pour laquelle l'antibiotique a un effet inhibant observable sur la croissance des bactéries. En se référant à la figure 16A, qui illustre la séquence $M(t_P)$ de la figure 14 en fonction du numéro $k$ des gouttelettes, on observe que la courbe $M(t_P)$ est sensiblement constante et égale à $\mu_{max}$ sur un intervalle $[1;N_0]$ avec $N_0 > N_g^{min}$ De manière analogue, la courbe $M(t_P)$ est sensiblement nulle sur un intervalle $[N_{CMI(t_P)}; N]$ avec $N_{CMI(t_P)} < N_g^{max}$ des gouttelettes de concentration initiale d'antibiotique $C_{max}$. L'intervalle $[N_0; N_{CMI(t_P)}]$ correspond donc à la zone de transition, la borne supérieure de cet intervalle correspondant à la concentration $N_{CMI(t_P)}$ recherchée.

L'identification de la zone de transition $[N_0; N_{CMI(tP)}]$ lors de l'étape **66** peut être réalisée par toute méthode mathématique connue, notamment tout méthode permettant d'identifier des points d'inflexion dans une courbe, et donc d'identifier deux points d'inflexion encadrant la zone de transition.

Par exemple, la courbe $M(t_P)$ est approximée par une fonction $\hat{f}(k)$ continue linéaire par morceau selon la relation :

$$\hat{f}(k) = \begin{cases} a.k + b & \forall k \in [1; N_0[ \\ \alpha.k + \beta & \forall k \in [N_0; N_{CMI(t_P)}] \\ c.k + d & \forall k \in ]N_{CMI(t_P)}; N] \end{cases}$$

où les valeurs des paramètres $N_0$, $\alpha$, $\beta$, $a$, $b$, $c$, $d$, et $N_{CMI(t_P)}$ sont calculées d'une manière connue en soi comme la solution optimale d'un problème d'optimisation minimisant une erreur d'estimation entre la séquence $M(t_P)$ et la séquence $\{\hat{f}(1), \hat{f}(2), ..., \hat{f}(k), ..., \hat{f}(N)\}$.

D'autres approximations de la séquence $M(t_P)$ sont possibles, par exemple une approximation polynomiale, obtenue notamment par la méthode des splines.

L'étape **64** se poursuit alors, en **70,** par la détermination, et la mémorisation, de la concentration initiale d'antibiotique correspondant au numéro de gouttelette $N_{CMI(tP)}$ selon la relation:

$$CMI(t_P) = [\widehat{ATB}]_{ini}(N_{CMI(t_P)})$$

Dans une étape **72** suivante, un test de stabilité de la concentration $CMI(t_P)$ est mis en œuvre. Le test consiste par exemple à vérifier si la séquence formée des concentrations $CMI(t_P)$ calculées pour $T$ derniers cycles de mesure de la fluorescence, par exemple les 3 derniers cycles, est stable. La concentration est jugée stable par exemple lorsqu'elle évolue de moins de $S\%$, par exemple 5%, sur les $T$ derniers instants de mesure. Les tests de stabilité permet notamment de stopper le processus au plus tôt et ne pas avoir à choisir un temps minimal d'incubation a priori.

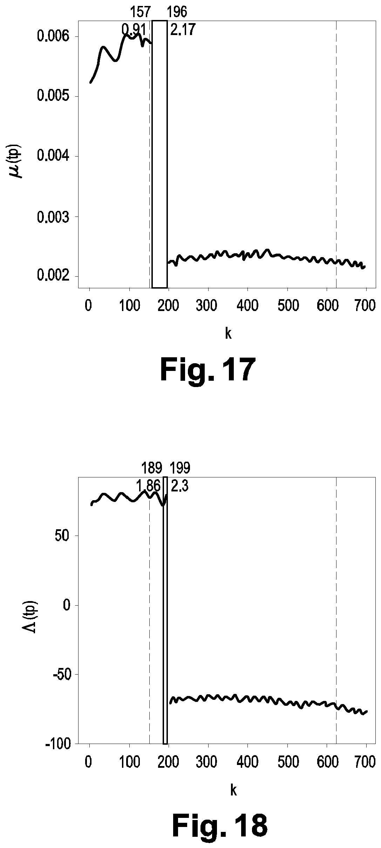

Les figures 17 et 18 illustrent le calcul de l'intervalle $[N_0; N_{CMI(t_P)}]$ respectivement pour les séquences $M(t_P)$ et $A(t_P)$ des figures 14 et 15. L'intervalle $[N_0; N_{CMI(t_P)}]$ déterminé sur la séquence $M(t_P)$ est égal à [157 ; 196], ce qui correspond à la gamme de concentration [0,97 ; 2,17]. L'intervalle $[N_0; N_{CMI(t_P)}]$ déterminé sur la séquence $A(t_P)$ est égal à [189 ; 199], ce qui correspond à la gamme de concentration [0,97 ; 2,3]. On note que les numéros $N_{CMI(tP)}$ déterminés pour les deux paramètres sont très proches (resp. 196 et 199). L'intervalle $[N_0; N_{CMI(t_P)}]$ est quant à lui déterminé avec plus de précision à l'aide de la séquence $A(t_P)$ dont la zone de transition est plus abrupte que la zone de transition de la séquence $M(t_P)$.

Si la concentration $CMI(t_P)$ n'est pas stable, l'étape **72** reboucle sur l'étape **66** pour calculer une concentration $CMI(t_P)$ en fonction des nouvelles mesures de fluorescence. Par contre, si la concentration $CMI(t_P)$ est stable, l'arrêt des mesures est alors commandé en **74.** La dernière concentration $CMI(t_P)$ calculée et mémorisée est alors la concentration inhibitrice minimale de l'antibiotique pour la bactérie objet des mesures.

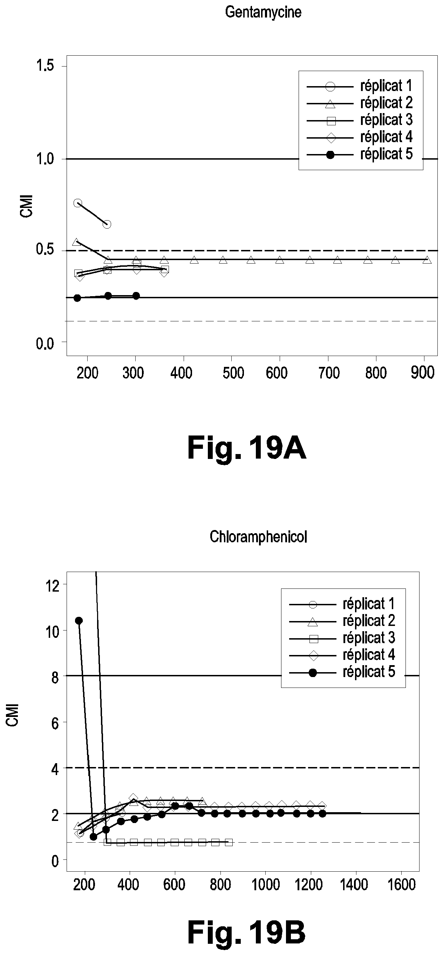

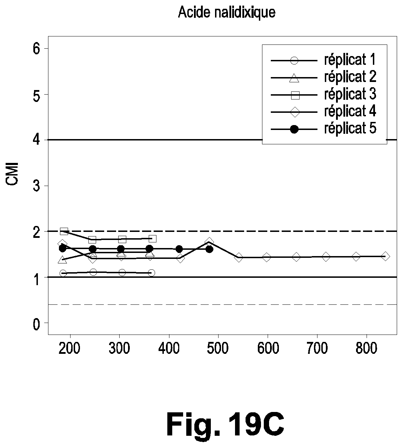

Les figures 19A à 19C illustrent les résultats du mode de réalisation venant d'être décrit. La production des mesures est celle décrite en relation avec les figures 6A à 6C. Plus particulièrement, les mesures décrites à ces figures font l'objet du traitement de données de l'étape de traitement **52** décrite précédemment en utilisant la séquence $M(t_P)$ pour le calcul de la concentration $CMI(t_P)$. Comme on peut le constater, la concentration $CMI(t_P)$ atteint rapidement une valeur stable comprise dans l'intervalle de tolérance de la CMI réglementaire. Concernant le réplica 3 de la figure 19B, la forme particulière de $CMI(t_P)$ provient d'une erreur de calibration de système de production de gouttelettes détecter a posteriori.

## VARIANTES

Il a été décrit un mode de réalisation particulier de l'invention. Evidemment, l'invention ne se limite pas à ce mode de réalisation. Notamment, les variantes suivantes, seules ou en combinaison, font partie de l'invention.

Le mode de réalisation est décrit en application à l'estimation d'une concentration minimale inhibitrice d'antibiotique de la croissance de bactéries et d'un intervalle de concentrations inhibitrices. L'invention s'applique également à la détermination d'autres grandeurs caractéristiques du pouvoir inhibiteur de l'antibiotique.

Il a été décrit un mode de réalisation particulier appliqué à l'analyse du pouvoir inhibiteur d'un antibiotique sur la croissance de bactérie. L'invention s'applique de la même manière à l'analyse du pouvoir inhibiteur de toute molécule sur un microorganisme, notamment l'analyse de l'effet inhibiteur d'un antifongique sur une moisissure, un champignon ou une levure.

Il a été décrit un mode de réalisation particulier dans lequel un seul type d'antibiotique est présent dans les échantillons. En variante, les échantillons peuvent comprendre un second antibiotique de concentration connue. Une étude des synergies des antibiotiques peut ainsi être menée. Par exemple, le procédé selon l'invention est mise en œuvre pour différentes concentrations du second antibiotique.

Il a été décrit un mode de réalisation dans lequel les bactéries sont initialement en nombre important pour éviter d'exacerber des particularités. En variante, un nombre réduit de bactérie, voire une seule bactérie, est présente dans les échantillons afin d'étudier celle-ci en particulier.

Il a été décrit un mode de réalisation dans lequel un gradient de concentration initial d'antibiotique est produit. En variante, la concentration de l'antibiotique est constante et un gradient de concentration de bactérie est produit. De manière générale, l'invention se rapporte ainsi à la formation d'un gradient d'une quantité initiale d'une molécule par microorganisme, entre une quantité minimale $Q_{min}$ et une quantité maximale $Q_{max}$.

Il a été décrit un gradient qui augmente linéairement depuis une valeur initiale jusqu'à une valeur finale. Un gradient linéaire permet de considérer chaque zone de concentration avec la même importance. D'autres types de gradient, notamment non linéaire, sont bien évidement possibles. Par exemple, des gradients par plateau où un nombre important de gouttelettes, par exemple quelques dizaines à une centaine, est généré pour un nombre limité de valeurs de concentrations, par exemple une dizaine, réparties sur la gamme $[C_{min}; C_{max}]$ de concentration d'antibiotique considéré. Avantageusement, ces valeurs de concentrations sont choisies en fonction des recommandations des instances réglementaires relatives à la mise en œuvre de la méthode de référence par micro-dilution telle que le CA-SFM (Comité d'Antibiogramme de la Société Française de Microbiologie) ou l'EUCAST (EUropean Committee on Antimicrobial Susceptibility Testing), de manière à réaliser en une seule expérience de multiples répétitions (quelques dizaines à une centaine, selon le nombre de gouttes par plateau) d'une expérience de micro-dilution.

Il a été décrit un traitement des mesures de fluorescence $x^k$. Bien évidemment, l'invention s'applique également à un traitement réalisé sur toute valeur déduite de manière bijective des mesures $x^k$, par exemple le nombre de bactéries qui se calcule en fonction de $x^k$ d'une manière connue en soi.

Il a été décrit le calcul de paramètres d'un modèle de croissance pour prendre en compte l'historique de croissance des bactéries dans la détermination d'une grandeur, par exemple la CMI.

En variante, l'historique est pris en compte en calculant une variation $V^k$ de la mesure $x^k$ en fonction du temps. Par exemple, cette variation $V^k(t_P)$ est égale à $(x^k(t_P^k) - x^k(t_{P-1}^k))$, ou égale à la moyenne $\frac{1}{P}\sum_p (x^k(t_p^k) - x^k(t_{p-1}^k))$, ou égale à $\max_p (x^k(t_p^k) - x^k(t_{p-1}^k))$. Le calcul de la $CMI(t_P)$ en fonction des $V^k(t_P)$ est réalisée d'une manière identique ou analogue à celle décrite en relation avec les valeurs $\mu^k(t_P)$ et $\lambda^k(t_P)$.

De même, il a été décrit la détermination de la grandeur en fonction d'un paramètre ($\mu^k(t_P)$ ou $\lambda^k(t_P)$). En variante, une grandeur, par exemple la CMI, peut être calculée pour chaque paramètre d'un ensemble de paramètres et la CMI finale calculée en fonction, ou sélectionnée parmi, les CMI calculées. Par exemple, la CMI finale est égale à la moyenne des CMI.

Il a été décrit un mode de réalisation dans lequel, la CMI est égale à la dernière valeur calculée jugée stable. En variante, le procédé se poursuit sur quelques cycles une fois la CMI ayant convergée et la CMI finale est calculée comme étant la moyenne des valeurs de CMI calculée une fois la convergence obtenue.

Il a été décrit un mode de réalisation utilisant l'analyseur décrit dans l'article *« Millifluidic droplet analyser for microbiology »*. Bien entendu, l'invention s'applique à tout type de dispositif et procédé produisant une pluralité d'échantillons présentant un gradient d'agent inhibiteur et/ou un gradient de microorganisme sensible audit agent. Notamment, l'invention s'applique par exemple à des échantillons n'ayant pas le même volume.

Il a été décrit la détermination d'une CMI, à savoir la CMI jugée comme réelle, celle-ci étant égale à la borne maximale de l'intervalle [$N_0$; $N_{CMI(t_P)}$]. Bien entendu, la CMI réglementaire, par exemple celle fixée par l'administration française ou étatsunienne, peut être également ou alternativement estimée à partir de cet intervalle. En effet, la détermination de l'intervalle étant stable, il est possible de déterminer une table de correspondance, ou toute autre règle de conversion appropriée, entre cet intervalle et la CMI réglementaire. A titre d'exemple, il est possible de déterminer si le microorganisme est susceptible, intermédiaire ou résistant à la molécule selon une classification réglementaire comparant une CMI aux concentrations critiques de la molécule supportable par l'homme. Une classification réglementaire de ce type est par exemple établie par le CA-SFM (Comité d'Antibiogramme de la Société Française de Microbiologie) ou l'EUCAST (EUropean Committee on Antimicrobial Susceptibility Testing).

## Revendications

1. Procédé d'estimation d'une grandeur $G_{inhib}$ quantifiant le pouvoir inhibiteur d'une molécule sur un microorganisme d'un type prédéterminé, comprenant :

   - la réalisation (50) d'une pluralité d'échantillons, comprenant chacun au moins un microorganisme dudit type, un milieu nutritif pour le microorganisme et une quantité initiale de la molécule par microorganisme dudit type présent dans l'échantillon, ladite quantité initiale étant croissante dans un intervalle [$Q_{min}$, $Q_{max}$] en fonction d'un classement prédéterminé des échantillons ;
   - l'incubation des échantillons;
   - pour chaque échantillon, la mesure (50) de la croissance des microorganismes dans l'échantillon en fonction du temps pendant une durée d'incubation prédéterminée ; et
   - la détermination (52) de la grandeur $G_{inhib}$ en fonction des mesures de la croissance des microorganismes dans les échantillons,

   *caractérisé* en ce que la détermination (52) de la grandeur $G_{inhib}$ comprend :

   - pour chaque échantillon, le calcul (66) d'une valeur reflétant la croissance du microorganisme dudit type à partir de mesures de croissance des microorganismes dans l'échantillon;
   - le classement (68) des valeurs calculées pour les échantillons en fonction du classement des échantillons ; et
   - la détermination (70) de la grandeur $G_{inhib}$ en fonction de l'évolution des valeurs classées,

2. Procédé selon la revendication 1, *caractérisé :*

   - **en ce que** la croissance du microorganisme est modélisée par un modèle de croissance en fonction du temps comprenant :

     ▪ une première phase de latence de durée $\lambda$ ;
     ▪ suivie d'une seconde phase de croissance exponentielle de pente maximale $\mu$ dans l'espace logarithmique ; et
     ▪ suivie d'une troisième phase stationnaire de valeur maximale A ;

   - et **en ce que** la valeur reflétant la croissance du microorganisme dudit type en fonction du temps est une estimation de la pente maximale $\mu$ et/ou une estimation de la durée de la phase de latente $\lambda$.

3. Procédé selon la revendication 1 ou 2, *caractérisé*

   - **en ce que** la grandeur $G_{inhib}$ comprend un intervalle $\left[Q_{min}^{MIC}, Q_{max}^{MIC}\right]$ pour lequel la croissance des microorganismes dans les échantillons est au moins partiellement inhibée ;
   - **en ce que** la quantité initiale de la molécule en fonction du classement des échantillons comprend :

     ▪ une première partie constante sur plusieurs échantillons et égale à $Q_{min}$, la borne inférieure $Q_{min}$ de l'intervalle [$Q_{min}$, $Q_{max}$] étant choisie de sorte qu'il n'y a aucune inhibition de la croissance des microorganismes dans les échantillons ;

EP 3 234 173 B1

- suivie d'une seconde partie strictement croissante de $Q_{min}$ à $Q_{max}$ ;
- suivie d'une troisième partie constante sur plusieurs échantillons et égale à $Q_{max}$, la borne supérieure $Q_{max}$ de l'intervalle $[Q_{min}, Q_{max}]$ étant choisie de sorte qu'il y a une inhibition totale de la croissance des microorganismes dans les échantillons ;

- et **en ce que** la détermination de l'intervalle $\left[Q_{min}^{MIC}, Q_{max}^{MIC}\right]$ comprend :

- l'identification d'une zone de transition dans l'évolution des valeurs classées entre deux zones extrêmes sensiblement stationnaires de ladite évolution; et
- la détermination de l'intervalle $\left[Q_{min}^{MIC}, Q_{max}^{MIC}\right]$ comme étant l'intervalle correspondant aux échantillons de la zone de transition identifiée.

4. Procédé selon la revendication 3, *caractérisé* **en ce que** l'identification de la zone de transition comprend la détermination deux points d'inflexion de l'évolution des valeurs classées, la zone de transition étant bornée par les deux points d'inflexion déterminés.

5. Procédé selon la revendication 3 ou 4, *caractérisé* **en ce que** l'indentification de la zone de transition comprend la modélisation de l'évolution des valeurs classées par une fonction continue linéaire par morceau comprenant uniquement deux segments de droite extrêmes et un segment de droite intermédiaire compris entre les deux segments de droite extrême, le segment de droite intermédiaire étant la zone de transition.

6. Procédé selon la revendication 3, 4 ou 5, *caractérisé* **en ce que** la grandeur $G_{inhib}$ comprend une quantité initiale minimale de molécules $Q_{MIC}$ qui est totalement inhibitrice de la croissance des microorganismes, et **en ce que** ladite quantité initiale minimale inhibitrice $Q_{MIC}$ étant choisie égale à la borne supérieure $Q_{max}^{MIC}$ de l'intervalle $\left[Q_{min}^{MIC}, Q_{max}^{MIC}\right]$.

7. Procédé selon l'une quelconque des revendications précédentes, *caractérisé* **en ce que** la borne inférieure $Q_{min}$ de l'intervalle $[Q_{min}, Q_{max}]$ est une quantité nulle de l'antibiotique.

8. Procédé selon l'une quelconque des revendications précédentes, *caractérisé :*

- **en ce que** les mesures de la croissance des bactéries dans les échantillons et la détermination de la grandeur $G_{inhib}$ en fonction desdites mesures sont mises en œuvre pour des durées d'incubation croissantes de manière à obtenir une séquence de grandeur $G_{inhib}$ en fonction de la durée d'incubation des échantillons ;
- **en ce que** le procédé comprend l'analyse de la stabilité de ladite séquence en fonction de la durée d'incubation; et
- **en ce que** la grandeur $G_{inhib}$ est la valeur de la séquence une fois la séquence stabilisée.

9. Procédé selon l'une quelconque des revendications précédentes, *caractérisé* **en ce que** les échantillons comprennent chacun initialement au moins 100 microorganismes, et de préférence au moins 500 microorganismes.

10. Procédé selon l'une quelconque des revendications précédentes, *caractérisé* **en ce que** les échantillons comprennent chacun une quantité initiale d'une deuxième molécule différente apte à inhiber la croissance des microorganismes, notamment une quantité initiale identique pour tous les échantillons.

11. Procédé selon l'une quelconque des revendications précédentes, *caractérisé* **en ce que** la quantité minimale de la molécule par microorganisme dudit type est une concentration de la molécule dans les échantillons, la concentration initiale de microorganisme dudit type dans les échantillons étant constante en fonction du classement des échantillons.

12. Procédé selon l'une quelconque des revendications précédentes, *caractérisé* **en ce que** le microorganisme est une bactérie, et **en ce que** la molécule est un antibiotique.

13. Procédé selon l'une quelconque des revendications 1 à 11, *caractérisé* **en ce que** le microorganisme est une levure ou une moisissure, et **en ce que** la molécule est un antifongique.

14. Procédé selon l'une quelconque des revendications précédentes, *caractérisé* **en ce que** le milieu nutritif comporte un élément métabolisable par le microorganisme sous la forme de molécule fluorescente, et **en ce que** la mesure de la croissance des microorganismes dans les échantillons est une mesure de la fluorescence des échantillons.

15. Procédé selon l'une quelconque des revendications 1 à 13, *caractérisé* **en ce que** l'absorbance des échantillons est variable en fonction de la quantité de microorganismes présents dans ceux-ci, et **en ce que** la mesure de la croissance des microorganismes dans les échantillons est une mesure de densité optique.

16. Procédé selon l'une quelconque des revendications précédentes, *caractérisé* **en ce que** la réalisation de la pluralité d'échantillon comporte la réalisation d'un train de gouttes formant échantillons dans de l'huile.

17. Dispositif d'estimation d'une grandeur $G_{inhib}$ quantifiant le pouvoir inhibiteur d'une molécule sur un microorganisme d'un type prédéterminé, comprenant :

- des moyens pour la réalisation d'une pluralité d'échantillons, comprenant chacun au moins un microorganisme dudit type, un milieu nutritif pour le microorganisme et une quantité initiale de la molécule par microorganisme dudit type présent dans l'échantillon, ladite quantité initiale étant croissante dans un intervalle [$Q_{min}$, $Q_{max}$] en fonction d'un classement prédéterminé des échantillons ;
- des moyens pour l'incubation des échantillons ;
- des moyens pour la mesure de la croissance des microorganismes dans chaque échantillon en fonction du temps pendant une durée d'incubation prédéterminée ; et
- des moyens de calcul configurés pour la détermination de la grandeur $G_{inhib}$ en fonction des mesures de la croissance des microorganismes dans les échantillons, *caractérisé* **en ce que** les moyens de calcul sont configurés pour mettre en œuvre :

- pour chaque échantillon, le calcul d'une valeur reflétant la croissance du microorganisme dudit type en fonction de la mesure de croissance des microorganismes dans l'échantillon;
- le classement des valeurs calculées pour les échantillons en fonction du classement des échantillons ; et
- la détermination de la grandeur $G_{inhib}$ en fonction de l'évolution des valeurs classées.

18. Dispositif selon la revendication 17, *caractérisé* **en ce qu'**il est configuré pour mettre en œuvre un procédé selon l'une quelconque des revendications 2 à 16.

**Patentansprüche**

1. Verfahren zur Schätzung einer Größe $G_{inhib}$, welche das Hemmvermögen eines Moleküls gegenüber einem Mikroorganismus vorbestimmter Art quantifiziert, wobei es Folgendes umfasst:

- Bereitstellen (50) einer Mehrzahl an Proben, wobei jede davon mindestens einen Mikroorganismus der betreffenden Art, ein Nährmedium für den Mikroorganismus sowie, pro Mikroorganismus der betreffenden Art, welcher in der Probe vorliegt, eine Ausgangsmenge des Moleküls umfasst, wobei diese innerhalb eines Intervalls [$Q_{min}$, $Q_{max}$] in Abhängigkeit von einer vorbestimmten Einstufung der Proben zunimmt;
- Inkubieren der Proben;
- für jede der Proben, Messen (50) des Wachstums der Mikroorganismen in der Probe in Abhängigkeit von der Zeit während einer vorbestimmten Dauer; und
- Bestimmen (52) der Größe $G_{inhib}$ in Abhängigkeit von den Messungen des Wachstums der Mikroorganismen in den Proben,

**dadurch *gekennzeichnet*, dass** die Bestimmung (52) der Größe $G_{inhib}$ Folgendes umfasst:

- für jede der Proben, Berechnen (66) eines Wertes, welcher das Wachstum des Mikroorganismus der betreffenden Art widerspiegelt, ausgehend von den Messungen des Wachstums der Mikroorganismen in der Probe;
- Einstufen (68) der Werte, wie sie für die Proben berechnet wurden, in Abhängigkeit von der Einstufung der Proben; und
- Bestimmen (70) der Größe $G_{inhib}$ in Abhängigkeit vom Verlauf der eingestuften Werte,

2. Verfahren nach Anspruch 1, *gekennzeichnet*:

- **dadurch, dass** das Wachstum des Mikroorganismus durch ein zeitabhängiges Wachstumsmodell modelliert wird, das Folgendes umfasst:

- eine erste Latenzphase einer Dauer $\lambda$;
- gefolgt von einer zweiten Phase des exponentiellen Wachstums mit einer maximalen Steigung $\mu$ im logarithmischen Raum; und
- gefolgt von einer dritten stationären Phase mit dem Höchstwert A;

- und dadurch, dass es sich bei dem Wert, welcher das Wachstum des Mikroorganismus der betreffenden Art widerspiegelt, um eine Schätzung der maximalen Steigung $\mu$ und/oder eine Schätzung der Dauer der Latenzphase $\lambda$ handelt.

3. Verfahren nach Anspruch 1 oder 2, *gekennzeichnet*

- **dadurch, dass** die Größe $G_{inhib}$ ein Intervall $\left[Q_{min}^{MIC}, Q_{max}^{MIC}\right]$ umfasst, in welchem das Wachstum der Mikroorganismen in den Proben zumindest teilweise gehemmt ist;
- dadurch, dass die Ausgangsmenge des Moleküls in Abhängigkeit von der Einstufung der Proben Folgendes umfasst:

- einen ersten Abschnitt, der über mehrere Proben konstant ist und gleich $Q_{min}$ ist, wobei der untere Grenzwert $Qmin$ des Intervalls [$Q_{min}$, $Q_{max}$] derart gewählt ist, dass keinerlei Hemmung des Wachstums der Mikroorganismen in den Proben vorliegt;
- gefolgt von einem zweiten Abschnitt, der von $Qmin$ bis $Qmax$ streng steigend ist;
- gefolgt von einem dritten Abschnitt, der über mehrere Proben konstant ist und gleich $Q_{max}$ ist, wobei der obere Grenzwert $Qmax$ des Intervalls [$Q_{min}$, $Q_{max}$] derart gewählt ist, dass keinerlei Hemmung des Wachstums der Mikroorganismen in den Proben vorliegt;

- und dadurch, dass die Bestimmung des Intervalls $\left[Q_{min}^{MIC}, Q_{max}^{MIC}\right]$ Folgendes umfasst:

- Identifizieren, im Verlauf der eingestuften Werte, eines Übergangsbereichs zwischen zwei im Wesentlichen stationären endständigen Bereichen des Verlaufs; und

- Bestimmen des Intervalls $\left[Q_{min}^{MIC}, Q_{max}^{MIC}\right]$ als dasjenige Intervall, welches den Proben des identifizierten Übergangsbereichs entspricht.

4. Verfahren nach Anspruch 3, **dadurch *gekennzeichnet*, dass** im Rahmen der Identifizierung des Übergangsbereichs zwei Wendepunkte des Verlaufs der eingestuften Werte bestimmt werden, wobei der Übergangsbereich von den beiden bestimmten Wendepunkten begrenzt wird.

5. Verfahren nach Anspruch 3 oder 4, **dadurch *gekennzeichnet*, dass** im Rahmen der Identifizierung des Übergangsbereichs der Verlauf der eingestuften Werte mittels einer stückweise linearen ununterbrochenen Funktion modelliert wird, die lediglich zwei endständige gerade Segmente und ein dazwischen liegendes gerades Segment umfasst, welches sich zwischen den beiden endständigen geraden Segmenten befindet, wobei es sich bei dem dazwischen liegenden geraden Segment um den Übergangsbereich handelt.

6. Verfahren nach Anspruch 3, 4 oder 5, **dadurch *gekennzeichnet*, dass** die Größe $G_{inhib}$ eine geringstmögliche Ausgangsmenge an Molekülen $Q_{MIC}$ umfasst, welche eine vollständig hemmende Wirkung auf das Wachstum der Mikroorganismen hat, und dadurch, dass die geringstmögliche hemmende Ausgangsmenge $Q_{MIC}$ derart gewählt ist, dass sie dem oberen Grenzwert $Q_{max}^{MIC}$ des Intervalls $\left[Q_{min}^{MIC}, Q_{max}^{MIC}\right]$ entspricht.

7. Verfahren nach einem beliebigen der vorhergehenden Ansprüche, **dadurch *gekennzeichnet*, dass** es sich bei dem unteren Grenzwert $Q_{min}$ des Intervalls [$Q_{min}$, $Q_{max}$] um eine Menge des Antibiotikums handelt, die gleich null ist.

8. Verfahren nach einem beliebigen der vorhergehenden Ansprüche, *gekennzeichnet:*

   - **dadurch, dass** die Messungen des Wachstums der Bakterien in den Proben und die Bestimmung der Größe $G_{inhib}$ in Abhängigkeit von den Messungen derart über zunehmende Inkubationsdauern durchgeführt werden, dass in Abhängigkeit von der Inkubationsdauer der Proben eine Größenreihe $G_{inhib}$ erhalten wird;
   - dadurch, dass im Rahmen des Verfahrens die Stabilität der Reihe in Abhängigkeit von der Inkubationsdauer untersucht wird; und
   - dadurch, dass es sich bei der Größe $G_{inhib}$ um den Wert der Reihe handelt, nachdem die Reihe stabilisiert wurde.

9. Verfahren nach einem beliebigen der vorhergehenden Ansprüche, **dadurch *gekennzeichnet,* dass** die Proben zu Beginn jeweils mindestens 100 Mikroorganismen, und vorzugsweise mindestens 500 Mikroorganismen, umfassen.

10. Verfahren nach einem beliebigen der vorhergehenden Ansprüche, **dadurch *gekennzeichnet,* dass** die Proben zu Beginn jeweils eine Ausgangsmenge eines zweiten Moleküls umfassen, welches dazu befähigt ist, das Wachstum der Mikroorganismen zu hemmen, insbesondere eine Ausgangsmenge, die bei sämtlichen Proben gleich groß ist.

11. Verfahren nach einem beliebigen der vorhergehenden Ansprüche, **dadurch *gekennzeichnet,* dass** es sich bei der geringsten Menge des Moleküls pro Mikroorganismus der betreffenden Art um eine Konzentration des Moleküls in den Proben handelt, wobei die Mikroorganismen-Anfangskonzentration der betreffenden Art in Abhängigkeit von der Einstufung der Proben konstant ist.

12. Verfahren nach einem beliebigen der vorhergehenden Ansprüche, **dadurch *gekennzeichnet,* dass** es sich bei dem Mikroorganismus um ein Bakterium handelt, und dadurch, dass es sich bei dem Molekül um ein Antibiotikum handelt.

13. Verfahren nach einem beliebigen der Ansprüche 1 bis 11, **dadurch *gekennzeichnet,* dass** es sich bei dem Mikroorganismus um eine Hefe oder einen Schimmelpilz handelt, und dadurch, dass es sich bei dem Molekül um ein Antimykotikum handelt.

14. Verfahren nach einem beliebigen der vorhergehenden Ansprüche, **dadurch *gekennzeichnet,* dass** das Nährmedium ein Element, welches von dem Mikroorganismus verstoffwechselt werden kann, in Form eines fluoreszierenden Moleküls aufweist, und dadurch, dass die Messung des Wachstums der Mikroorganismen in den Proben einer Messung der Fluoreszenz der Proben entspricht.

15. Verfahren nach einem beliebigen der Ansprüche 1 bis 13, **dadurch *gekennzeichnet,* dass** sich der Absorptionswert der Proben in Abhängigkeit von der Menge an Mikroorganismen ändert, welche darin vorliegen, und dadurch, dass die Messung des Wachstums der Mikroorganismen in den Proben einer Messung der optischen Dichte entspricht.

16. Verfahren nach einem beliebigen der vorhergehenden Ansprüche, **dadurch *gekennzeichnet,* dass** im Rahmen des Bereitstellens der Mehrzahl an Proben eine Aneinanderreihung von Tropfen bereitgestellt wird, die Proben in Öl bilden.

17. Vorrichtung zur Schätzung einer Größe $G_{inhib}$, welche das Hemmvermögen eines Moleküls gegenüber einem Mikroorganismus vorbestimmter Art quantifiziert, wobei sie Folgendes umfasst:

    - Mittel zum Bereitstellen einer Mehrzahl an Proben, wobei jede davon mindestens einen Mikroorganismus der betreffenden Art, ein Nährmedium für den Mikroorganismus sowie, pro Mikroorganismus der betreffenden Art, welcher in der Probe vorliegt, eine Ausgangsmenge des Moleküls umfasst, wobei diese innerhalb eines Intervalls $[Q_{min}, Q_{max}]$ in Abhängigkeit von einer vorbestimmten Einstufung der Proben zunimmt;
    - Mittel zum Inkubieren der Proben;
    - Mittel zum Messen des Wachstums der Mikroorganismen in jeder der Proben in Abhängigkeit von der Zeit während einer vorbestimmten Dauer; und
    - Rechenmittel, welche dafür ausgelegt sind, die Größe $G_{inhib}$ in Abhängigkeit von den Messungen des Wachstums der Mikroorganismen in den Proben zu bestimmen, **dadurch *gekennzeichnet,* dass** die Rechenmittel dafür ausgelegt sind, Folgendes durchzuführen:

      - für jede der Proben, Berechnen eines Wertes, welcher das Wachstum des Mikroorganismus der betreffenden Art widerspiegelt, in Abhängigkeit von der Messung des Wachstums der Mikroorganismen in der Probe;
- Einstufen der Werte, welche für die Proben berechnet wurden, in Abhängigkeit von der Einstufung der Proben; und
- Bestimmen der Größe $G_{inhib}$ in Abhängigkeit vom Verlauf der eingestuften Werte.

18. Vorrichtung nach Anspruch 17, **dadurch *gekennzeichnet,* dass** sie dafür ausgelegt ist, ein Verfahren nach einem beliebigen der Ansprüche 2 bis 16 durchzuführen.

**Claims**

1. Method for estimating a quantity $G_{inhib}$ quantifying the inhibitory capacity of a molecule on a microorganism of a predetermined type, comprising:

   - preparing (50) a plurality of samples, each comprising at least one microorganism of said type, a nutrient medium for the microorganism and an initial amount of the molecule per microorganism of said type present in the sample, said initial amount increasing in a range [$Q_{min}$, $Q_{max}$] as a function of a predetermined classification of the samples;
   - incubating the samples;
   - for each sample, measuring (50) the growth of the microorganisms in the sample as a function of time for a predetermined incubation time; and
   - determining (52) the quantity $G_{inhib}$ as a function of the measurements of the growth of the microorganisms in the samples,

   **characterized** in that the determination (52) of the quantity $G_{inhib}$ comprises:

   - for each sample, calculation (66) of a value reflecting the growth of the microorganism of said type based on measurements of growth of the microorganisms in the sample;
   - classification (68) of the values calculated for the samples as a function of the classification of the samples; and
   - determination (70) of the quantity $G_{inhib}$ as a function of the variation of the classified values.

2. Method according to Claim 1, **characterized:**

   - **in that** the growth of the microorganism is modeled by a model of growth as a function of time comprising:

     - a first lag phase of duration $\lambda$;
     - followed by a second exponential growth phase of maximum slope $\mu$ in the logarithmic space; and
     - followed by a third stationary phase of maximum value A;

   - and **in that** the value reflecting the growth of the microorganism of said type as a function of time is an estimate of the maximum slope $\mu$ and/or an estimate of the duration of the lag phase $\lambda$.

3. Method according to Claim 1 or 2, **characterized**

   - **in that** the quantity $G_{inhib}$ comprises a range $\left[Q_{min}^{MIC}, Q_{max}^{MIC}\right]$ for which the growth of the microorganisms in the samples is at least partially inhibited;
   - **in that** the initial amount of the molecule as a function of the classification of the samples comprises:

     - a first part that is constant for several samples and equal to $Q_{min}$, the lower limit $Q_{min}$ of the range [$Q_{min}$, $Q_{max}$] being selected so that there is no inhibition of the growth of the microorganisms in the samples;
     - followed by a second part strictly increasing from $Q_{min}$ to $Q_{max}$;
     - followed by a third part that is constant for several samples and equal to $Q_{max}$, the upper limit $Q_{max}$ of the range [$Q_{min}$, $Q_{max}$] being selected so that there is complete inhibition of the growth of the microorganisms in the samples;

   - and **in that** determination of the range $\left[Q_{min}^{MIC}, Q_{max}^{MIC}\right]$ comprises:

- identifying a transition zone in the variation of the classified values between two roughly stationary extreme zones of said variation; and
- determining the range $\left[Q_{min}^{MIC}, Q_{max}^{MIC}\right]$ as being the range corresponding to the samples of the transition zone identified.

4. Method according to Claim 3, *characterized* **in that** identification of the transition zone comprises determining two inflexion points of the variation of the classified values, the transition zone being bounded by the two inflexion points determined.

5. Method according to Claim 3 or 4, *characterized* **in that** identification of the transition zone comprises modeling the variation of the classified values by a piecewise linear continuous function comprising only two extreme straight-line segments and an intermediate straight-line segment between the two extreme straight-line segments, the intermediate straight-line segment being the transition zone.

6. Method according to Claim 3, 4 or 5, *characterized* **in that** the quantity $G_{inhib}$ comprises a minimum initial quantity of molecules $Q_{MIC}$ that completely inhibits the growth of the microorganisms, and **in that** said initial minimum inhibitory amount $Q_{MIC}$ is selected equal to the upper limit $Q_{max}^{MIC}$ of the range $\left[Q_{min}^{MIC}, Q_{max}^{MIC}\right]$.

7. Method according to any one of the preceding claims, *characterized* **in that** the lower limit $Q_{min}$ of the range [$Q_{min}$, $Q_{max}$] is a zero amount of the antibiotic.

8. Method according to any one of the preceding claims, *characterized:*

   - **in that** the measurements of the growth of the bacteria in the samples and determination of the quantity $G_{inhib}$ as a function of said measurements are performed for increasing incubation times so as to obtain a sequence of quantities $G_{inhib}$ as a function of the incubation time of the samples;
   - **in that** the method comprises analysis of the stability of said sequence as a function of the incubation time; and
   - **in that** the quantity $G_{inhib}$ is the value of the sequence once the sequence has stabilized.

9. Method according to any one of the preceding claims, *characterized* **in that** the samples each comprise initially at least 100 microorganisms, and preferably at least 500 microorganisms.

10. Method according to any one of the preceding claims, *characterized* **in that** the samples each comprise an initial amount of a second different molecule able to inhibit the growth of the microorganisms, notably an identical initial amount for all the samples.

11. Method according to any one of the preceding claims, *characterized* **in that** the minimum amount of the molecule per microorganism of said type is a concentration of the molecule in the samples, the initial concentration of microorganism of said type in the samples being constant as a function of the classification of the samples.

12. Method according to any one of the preceding claims, *characterized* **in that** the microorganism is a bacterium, and **in that** the molecule is an antibiotic.

13. Method according to any one of Claims 1 to 11, *characterized* **in that** the microorganism is a yeast or a mold, and **in that** the molecule is an antifungal.

14. Method according to any one of the preceding claims, *characterized* **in that** the nutrient medium comprises an element that can be metabolized by the microorganism in the form of a fluorescent molecule, and **in that** the measurement of the growth of the microorganisms in the samples is a measurement of the fluorescence of the samples.

15. Method according to any one of Claims 1 to 13, *characterized* **in that** the absorbance of the samples is variable as a function of the quantity of microorganisms present in the latter, and **in that** the measurement of the growth of the microorganisms in the samples is a measurement of optical density.

**16.** Method according to anyone of the preceding claims, ***characterized*** **in that** producing the plurality of samples comprises preparation of a train of droplets forming samples in oil.

**17.** Device for estimating a quantity $G_{inhib}$ quantifying the inhibitory capacity of a molecule on a microorganism of a predetermined type, comprising:

- means for preparing a plurality of samples, each comprising at least one microorganism of said type, a nutrient medium for the microorganism and an initial amount of the molecule per microorganism of said type present in the sample, said initial amount increasing in a range [$Q_{min}$, $Q_{max}$] as a function of a predetermined classification of the samples;
- means for incubating the samples;
- means for measuring the growth of the microorganisms in each sample as a function of time for a predetermined incubation time; and
- calculating means configured for determining the quantity $G_{inhib}$ as a function of the measurements of the growth of the microorganisms in the samples,

***characterized*** **in that** the calculating means are configured to perform:

- for each sample, calculation of a value reflecting the growth of the microorganism of said type as a function of the measurement of growth of the microorganisms in the sample;
- classification of the values calculated for the samples as a function of the classification of the samples; and
- determination of the quantity $G_{inhib}$ as a function of the variation of the classified values.

**18.** Device according to Claim 17, **characterized in that** it is configured for carrying out a method according to any one of Claims 2 to 16.

**Fig. 1**

**Fig. 2**

$G_{k+1}$     $G_k$     $G_{k-1}$     26

35

**Fig. 3A**

$I^{k+1}(t_p^{k+1})$     $I^k(t_p^k)$     $I^{k-1}(t_p^{k-1})$

signal de sortie (volts)

temps d'acquisition (secondes)

**Fig. 3B**

temps d'acquisition: 0 min

sortie (volt)

$[ATB]_{ini} \geq CMI$ ("sub CMI")     $[ATB]_{ini} < CMI$

**Fig. 4A**

temps d'acquisition: 400 min

sortie (volt)

sub-CMI

CMI

numéro des gouttelettes k

$Q_{MIC}$

**Fig. 4B**

**Fig. 5**

**Fig. 6A**

Chloramphenicol

## Fig. 6B

Acide nalidixique

## Fig. 6C

## Fig. 7

```
┌─────────────────────────────────────────┐
│  54 ─┐  ┌──────────────────────────┐     │
│      │  │ Définition des paramètres │     │
│ 50 ─┘   │ pour la production des    │     │
│         │      échantillons         │     │
│         └──────────────────────────┘     │
│                    │                       │
│                    ▼                       │
│         ┌──────────────────────────┐     │
│  56 ─── │ Définition des consignes  │     │
│         │  de débits des seringues  │     │
│         └──────────────────────────┘     │
│                    │                       │
│                    ▼                       │
│         ┌──────────────────────────┐     │
│  58 ─── │     Production des         │     │
│         │      échantillons          │     │
│         └──────────────────────────┘     │
│                    │                       │
│                    ▼                       │
│         ┌──────────────────────────┐     │
│  60 ─── │  Mesure en fonction du     │◄───│
│         │ temps de la fluorescence   │     │
│         │    des échantillons        │     │
│         └──────────────────────────┘     │
└─────────────────────────────────────────┘
```

Calcul de la pente $\mu$ et/ou de la latence $\lambda$ pour la nouvelle acquisition — 66

Calcul d'une zone de transition de la pente $\mu$ et/ou de la latence $\lambda$ Calcul de la position de la CMI — 68

74

Arrêt de la mesure

62

Estimation concentration réelle de l'antibiotique

Calcul CMI — 70

72

non    CMI stable ?    oui

64

52

FIN

**Fig. 8**

**Fig. 9**

**Fig. 10**

**Fig. 11**

**Fig. 12A**

**Fig. 12B**

**Fig. 13**

**Fig. 14**

**Fig. 15**

M(tp)

$\mu_{max}$

$\mu_{min}=0$

k

$C_{min}$ | antibiotique gradient | $C_{max}$

pas d'effet inhibiteur

estimation CMI(tp)

inhibition totale

**Fig. 16A**

$\Rightarrow$

M(tp), $\hat{f}(k)$

k

$C_{min}$ | antibiotique gradient | $C_{max}$

estimation CMI(tp)

**Fig. 16B**

Fig. 17

Fig. 18

Gentamycine

## Fig. 19A

Chloramphenicol

## Fig. 19B

Fig. 19C

# RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2008107881 A
- US 2012077206 A
- WO 2014155020 A
- FR 2916761

**Littérature non-brevet citée dans la description**

- **DE L. BARABAN et al.** Millifluidic droplet analyser for microbiology. *Lab on Chip,* 2011, vol. 11, 4057
- Modélisation et microbiologie prévisionnelle : élaboration d'un nouvel outil pour l'agroalimentaire. **ROSSO L.** Thèse de doctorat. Université Claude Bernard Lyon 1, 1995
- **KAHM M. et al.** grofit : Fitting Biological Growthe Curve with R. *Journal of Statistical Software,* Février 2010, vol. 33 (7